(19) Europäisches Patentamt
European Patent Office
Office européen des brevets

(11) Publication number : **0 427 445 B1**

(12) **EUROPEAN PATENT SPECIFICATION**

(45) Date of publication of patent specification : **08.06.94 Bulletin 94/23**

(51) Int. Cl.$^5$ : **C07C 251/00,** C07C 327/42, A01N 37/36

(21) Application number : **90311904.8**

(22) Date of filing : **30.10.90**

(54) **Benzylideneaminoxyalkanoic acid (thio) amide derivative, process for preparing the same and herbicide.**

(30) Priority : **09.11.89 JP 289950/89**
**09.03.90 JP 56662/90**

(43) Date of publication of application : **15.05.91 Bulletin 91/20**

(45) Publication of the grant of the patent : **08.06.94 Bulletin 94/23**

(84) Designated Contracting States : **DE ES FR GB IT**

(56) References cited :
**EP-A- 0 023 890**
**DD-A- 146 593**
**GB-A- 1 080 879**

(73) Proprietor : **UBE INDUSTRIES, LTD.**
**12-32, Nishihonmachi 1-chome**
**Ube-shi, Yamaguchi-ken (JP)**

(72) Inventor : **Harada, Katsumasa, c/o Ube Research Laboratory**
**Ube Industries Ltd.**
**1978-5, Oaza Kogushi**
**Ube-shi, Yamaguchi-ken (JP)**

Inventor : **Akiyoshi, Yuji, c/o Ube Research Laboratory**
**Ube Industries Ltd.**
**1978-5, Oaza Kogushi**
**Ube-shi, Yamaguchi-ken (JP)**

Inventor : **Abe, Takaaki, c/o Ube Research Laboratory**
**Ube Industries Ltd.**
**1978-5, Oaza Kogushi**
**Ube-shi, Yamaguchi-ken (JP)**

Inventor : **Shiraishi, Hiroshi, c/o Ube Research Laboratory**
**Ube Industries Ltd.**
**1978-5, Oaza Kogushi**
**Ube-shi, Yamaguchi-ken (JP)**

Inventor : **Yamamoto, Kaoru, c/o Ube Research Laboratory**
**Ube Industries Ltd.**
**1978-5, Oaza Kogushi**
**Ube-shi, Yamaguchi-ken (JP)**

Inventor : **Hayama, Takashi, c/o Ube Research Laboratory**
**Ube Industries Ltd.**
**1978-5, Oaza Kogushi**
**Ube-shi, Yamaguchi-ken (JP)**

Inventor : **Shiraishi, Ikuo, c/o Ube Research Laboratory**
**Ube Industries Ltd.**
**1978-5, Oaza Kogushi**
**Ube-shi, Yamaguchi-ken (JP)**

(74) Representative : **Green, Mark Charles et al**
**Urquhart-Dykes & Lord,**
**91 Wimpole Street**
**London W1M 8AH (GB)**

**EP 0 427 445 B1**

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue Saint-Denis, 75001 PARIS

## Description

This invention relates to a benzylideneaminoxyalkanoic acid (thio)amide derivative which is available as a herbicide, a process for preparing the same and use thereof.

It has heretofore been known that many kinds of phenoxyalkanoic acid amide derivatives as a component of a herbicidal composition (for example, Japanese Provisional Patent Publications No. 14505/1982, No. 14506/1982, No. 67653/1983, No. 113155/1983, No. 113156/1983, No. 29645/1984 and No. 184105/1984), but it has been not known concerning a benzylideneaminoxyalkanoic acid (thio)amide derivative

EP-A-0023890 discloses herbicidal compounds having the formula

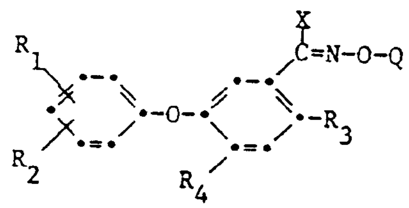

wherein

$R_1$ is hydrogen, nitro, cyano or trifluoromethyl,

$R_2$ and $R_3$ are hydrogen, halogen, nitro or cyano,

$R_4$ is hydrogen or halogen,

X is hydrogen, halogen, cyano, nitro, lower alkyl, lower alkanoyl, carboxylic acid lower alkyl ester or carboxylic acid amide, and

Q is widely defined, but includes amido-substituted alkyl.

An object of the present invention is to provide a herbicidal compound which shows wide herbicidal effects against various grass weeds and broadleaved weeds, and has high activities against weeds which are hardly removed by the conventional herbicides while it has no chemical damage against young seedling paddy rice plant, wheat, soybean, corn and the like with high selectivity.

The benzylideneaminoxyalkanoic acid (thio)amide derivative of the present invention is shown by the following formula:

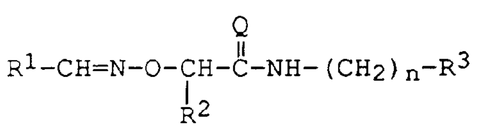

(I)

wherein Q represents an oxygen atom or a sulfur atom, n is an integer of 0 or 1, and when Q is an oxygen atom, $R^1$ represents a phenyl group which may have one or two substituents selected from the group consisting of a lower alkyl group, a lower alkoxy group, a halogen atom, a halo-lower alkyl group, a halo-lower alkoxy group, a halo-lower alkylthio group, a halo-lower alkylsulfonyl group, a cyano group and a nitro group, or a thienyl group or a furyl group which may have a substituent selected from a lower alkyl group or a halogen atom; $R^2$ represents a lower alkyl group; and $R^3$ represents a phenyl group which may have one or two substituents selected from the group consisting of a lower alkyl group, a halogen atom, a halo-lower alkyl group and a lower alkoxy group, or a cycloalkyl group, a thienyl group or a furyl group; and when Q represents a sulfur atom, $R^1$ represents a phenyl group which may be substituted by a halogen atom, a halo-lower alkyl group, a halo-lower alkoxy group, a cyano group or a nitro group; $R^2$ represents a lower alkyl group and $R^3$ represents a phenyl group which may be substituted by a halogen atom or a thienyl group.

Also, the process for preparing the benzylideneaminoxyalkanoic acid amide derivative of the present invention comprises reacting an oxime compound represented by the formula:

$$R^1\text{-CH=N-OH} \qquad \text{(II)}$$

wherein $R^1$ has the same meaning as defined above, with 2-halogenoalkanoic acid amide represented by the formula:

$$X-\underset{\mid \atop R^2}{CH}-\overset{O \atop \|}{C}-NH-(CH_2)_n-R^3 \qquad (III)$$

wherein $R^2$, $R^3$ and n have the same meanings as defined above and X represents a halogen atom, or reacting a benzylideneaminoxyalkanoic acid halide represented by the formula:

$$R^1-CH=N-O-\underset{\mid \atop R^2}{CH}-\overset{O \atop \|}{C}-Y \qquad (IV)$$

wherein $R^1$ and $R^2$ have the same meanings as defined above and Y represents a halogen atom, with an amine represented by the formula:

$$NH_2-(CH_2)_n-R^3 \qquad (V)$$

wherein $R^3$ and n have the same meanings as defined above, or reacting an aldehyde represented by the formula:

$$R^1-CHO \qquad (VI)$$

wherein $R^1$ has the same meaning as defined above, with an aminoxyalkanoic acid amide represented by the formula:

$$NH_2-O-\underset{\mid \atop R^2}{CH}-\overset{O \atop \|}{C}-NH-(CH_2)_n-R^3 \qquad (VII)$$

wherein $R^2$, $R^3$ and n have the same meanings as defined above, or reacting a benzylideneamonoxyalkanoic acid ester represented by the formula:

$$R^1-CH=N-O-\underset{\mid \atop R^2}{CH}-\overset{O \atop \|}{C}-OZ \qquad (VIII)$$

wherein $R^1$ and $R^2$ have the same meanings as defined above, and Z represents a lower alkyl group, with an amine represented by the formula:

$$NH_2-(CH_2)_n-R^3 \qquad (IX)$$

wherein $R^3$ and n have the same meanings as defined above.

Also, the process for preparing the benzylideneaminoxyalkanoic acid thioamide derivative of the present invention comprises reacting a compound represented by the formula:

$$R^1-CH=N-O-\underset{\mid \atop R^2}{CH}-\overset{O \atop \|}{C}-NH-(CH_2)_n-R^3 \qquad (I')$$

wherein $R^1$, $R^2$, $R^3$ and n have the same meanings as defined above, with a thiocarbonylation reagent.

Further, a herbicide of the present invention comprises the above benzylideneaminoxyalkanoic acid (thio)amide derivative as an active ingredient.

In the following, the present invention is explained in more detail.

Among the benzylideneaminoxyalkanoic acid (thio)amide derivatives of the present invention, the benzylideneaminoxyalkanoic acid amide derivative (Q = O) is represented by the following formula:

$$R^1-CH=N-O-\underset{\underset{R^2}{|}}{CH}-\overset{\overset{O}{\|}}{C}-NH-(CH_2)_n-R^3 \qquad (I')$$

wherein $R^1$, $R^2$, $R^3$ and n have the same meanings as defined above.

In the above formula, the lower alkyl group as the substituents for the phenyl group, the thienyl group and the furyl group may be mentioned, for example, a methyl group, an ethyl group, a propyl group, an isopropyl group, a butyl group and a t-butyl group, and a methyl group is particularly preferred. The lower alkoxy group as the substituent may be mentioned, for example, a methoxy group and an ethoxy group, and a methoxy group is particularly preferred. The halogen atom as the substituent may include a chlorine atom, a bromine atom and a fluorine atom, and a chlorine atom and a fluorine atom are particularly preferred. The halo-lower alkyl group as the substituent may include a trifluoromethyl group, a difluoromethyl group, a 1,1-difluoroethyl group, a 1,1,2,2-tetrafluoroethyl group and a pentafluoroethyl group, and a trifluoromethyl group is particularly preferred. The halo-lower alkoxy group as the substituent may include a difluoromethoxy group, a trifluoromethoxy group and a 1,1,2-trifluoro-2-chloroethoxy group, and a trifluoromethoxy group is particularly preferred. The halo-lower alkylthio group as the substituent may include a trifluoromethylthio group, a difluorochloromethylthio group and a difluorobromomethylthio group. The halo-lower alkylsulfonyl group as the substituent may include a trifluoromethylsulfonyl group. As the lower alkyl group of $R^2$, there may be mentioned a methyl group and an ethyl group, and a ethyl group is particularly preferred.

In the above formula (I′), preferred compounds are those wherein $R^1$ is a phenyl group substituted by a chlorine atom, a fluorine atom, a methyl group, a methoxy group, a trifluoromethyl group, a trifluoromethoxy group, a cyano group or a nitro group, $R^2$ is an ethyl group, and $R^3$ is a phenyl group which may be substituted by a methyl group, a halogen atom or a trifluoromethyl group, or a thienyl group.

Preferred compounds included in the formula (I′) are enumerated in Table 1.

Table 1

$R^1-CH=N-O-\underset{\displaystyle R^2}{\underset{|}{CH}}-CONH-(CH_2)_n-R^3$

| No. | $R^1$ | $R^2$ | $R^3$ | n | Physical property |
|---|---|---|---|---|---|
| 1 | 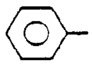 | Et | 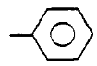 | 1 | m.p. 53~55℃ |
| 2 | 〃 | 〃 | 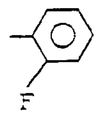 | 1 | m.p. 86~87℃ |
| 3 | 〃 | 〃 | 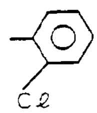 | 1 | $n_D^{29.4}$ 1.5746 |
| 4 | 〃 | 〃 | 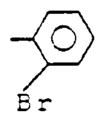 | 1 | m.p. 96~98℃ |
| 5 | 〃 | 〃 | 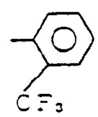 | 1 | m.p. 74~76℃ |
| 6 | 〃 | 〃 | 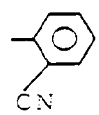 | 1 | |
| 7 | 〃 | 〃 | 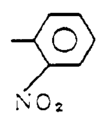 | 1 | |
| 8 | 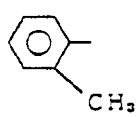 | 〃 | 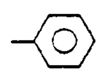 | 1 | m.p. 60~63℃ |
| 9 | 〃 | 〃 | 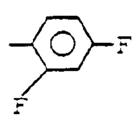 | 0 | $n_D^{25.2}$ 1.5501 |
| 10 | 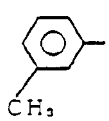 | 〃 | 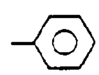 | 1 | $n_D^{29.6}$ 1.5632 |
| 11 | 〃 | 〃 | 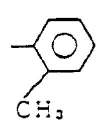 | 1 | m.p. 81~83℃ |

| No. | $R^1$ | $R^2$ | $R^3$ | n | Physical property |
|---|---|---|---|---|---|
| 12 | 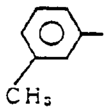 | Et | 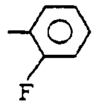 | 1 | m.p. 62～64 ℃ |
| 13 | 〃 | 〃 | 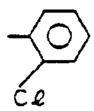 | 1 | m.p. 67～70 ℃ |
| 14 | 〃 | 〃 | 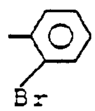 | 1 | |
| 15 | 〃 | 〃 | 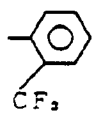 | 1 | $n_D^{26.9}$ 1.5328 |
| 16 | 〃 | 〃 | 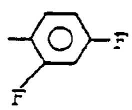 | 0 | $n_D^{27.0}$ 1.5488 |
| 17 | 〃 | 〃 | 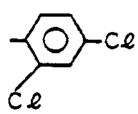 | 0 | |
| 18 | 〃 | 〃 | 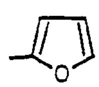 | 1 | $n_D^{20.4}$ 1.5419 |
| 19 | 〃 | 〃 | 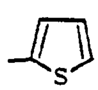 | 1 | $n_D^{23.5}$ 1.5763 |
| 20 | 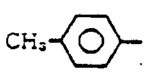 | 〃 | 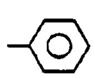 | 1 | m.p. 72～74 ℃ |
| 21 | 〃 | 〃 | 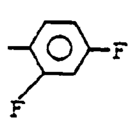 | 0 | m.p. 50～52 ℃ |

| No. | $R^1$ | $R^2$ | $R^3$ | n | Physical property |
|---|---|---|---|---|---|
| 22 | 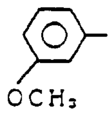 | Et | 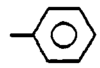 | 1 | $n_D^{21.6}$ 1.5726 |
| 23 | 〃 | 〃 | 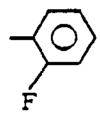 | 1 | $n_D^{24.8}$ 1.5594 |
| 24 | 〃 | 〃 | 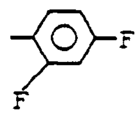 | 0 | $n_D^{27.6}$ 1.5552 |
| 25 | 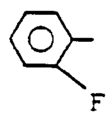 | 〃 | 〃 | 0 | m.p. 49～51℃ |
| 26 | 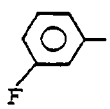 | 〃 | 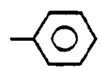 | 1 | m.p. 75～77℃ |
| 27 | 〃 | 〃 | 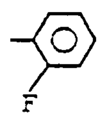 | 1 | m.p. 56～58℃ |
| 28 | 〃 | 〃 | 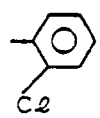 | 1 | m.p. 98～99℃ |
| 29 | 〃 | 〃 | 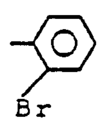 | 1 | |
| 30 | 〃 | 〃 | 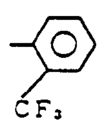 | 1 | m.p. 61～63℃ |
| 31 | 〃 | 〃 | 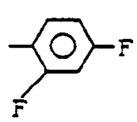 | 0 | m.p. 53～55℃ |

| No. | R¹ | R² | R³ | n | Physical property |
|---|---|---|---|---|---|
| 32 | 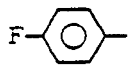 | Et | 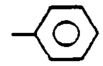 | 1 | m.p. 65~67℃ |
| 33 | 〃 | 〃 | 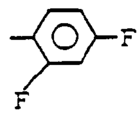 | 0 | $n_D^{25.2}$ 1.5305 |
| 34 | 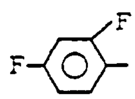 | 〃 | 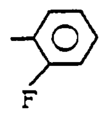 | 1 | $n_D^{27.0}$ 1.5472 |
| 35 | 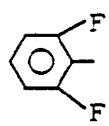 | 〃 | 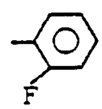 | 1 | $n_D^{27.0}$ 1.5414 |
| 36 | 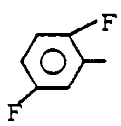 | 〃 | 〃 | 1 | m.p. 69~70℃ |
| 37 | 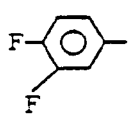 | 〃 | 〃 | 1 | m.p. 82~83℃ |
| 38 | 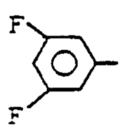 | 〃 | 〃 | 1 | m.p. 75~77℃ |
| 39 | 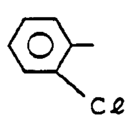 | 〃 | 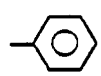 | 1 | m.p. 100~101℃ |
| 40 | 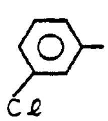 | 〃 | 〃 | 1 | m.p. 75~78℃ |
| 41 | 〃 | 〃 | 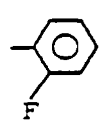 | 1 | m.p. 73~74℃ |
| 42 | 〃 | 〃 | 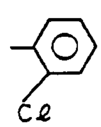 | 1 | m.p. 83~85℃ |

| No. | R¹ | R² | R³ | n | Physical property |
|---|---|---|---|---|---|
| 43 | 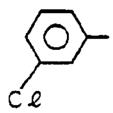 | Et | 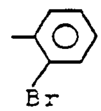 | 1 | m.p. 97～99℃ |
| 44 | 〃 | 〃 | 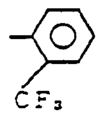 | 1 | m.p. 57～59℃ |
| 45 | 〃 | 〃 | 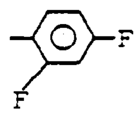 | 0 | m.p. 73～75℃ |
| 46 | 〃 | 〃 | 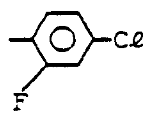 | 0 | |
| 47 | 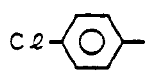 | 〃 | 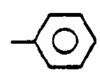 | 1 | m.p. 83～85℃ |
| 48 | 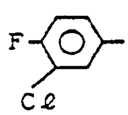 | 〃 | 〃 | 1 | $n_D^{29.8}$ 1.5633 |
| 49 | 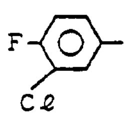 | 〃 | 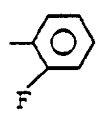 | 1 | $n_D^{29.8}$ 1.5540 |
| 50 | 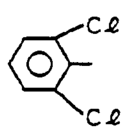 | 〃 | 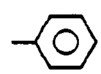 | 1 | m.p. 75～76℃ |
| 51 | 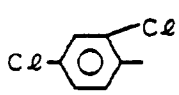 | 〃 | 〃 | 1 | m.p. 82～84℃ |
| 52 | 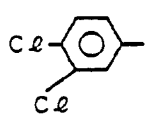 | 〃 | 〃 | 1 | $n_D^{26.8}$ 1.5762 |
| 53 | 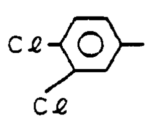 | 〃 | 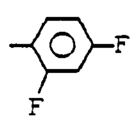 | 0 | $n_D^{27.4}$ 1.5731 |

| No. | $R^1$ | $R^2$ | $R^3$ | n | Physical property |
|---|---|---|---|---|---|
| 54 | 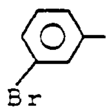 | Et | 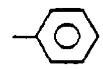 | 1 | m.p. 90～91℃ |
| 55 | 〃 | 〃 | 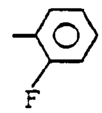 | 1 | m.p. 82～83℃ |
| 56 | 〃 | 〃 | 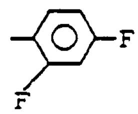 | 0 | $n_D^{26.4}$ 1.5650 |
| 57 | 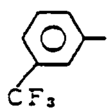 | Me | 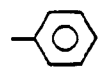 | 1 | m.p. 64～66℃ |
| 58 | 〃 | Et | 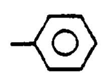 | 1 | $n_D^{25.6}$ 1.5321 |
| 59 | 〃 | 〃 | 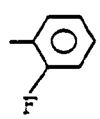 | 1 | m.p. 57～58℃ |
| 60 | 〃 | 〃 | 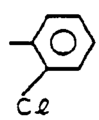 | 1 | $n_D^{24.9}$ 1.5383 |
| 61 | 〃 | 〃 | 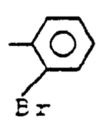 | 1 | m.p. 65～66℃ |
| 62 | 〃 | 〃 | 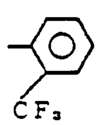 | 1 | $n_D^{20.8}$ 1.4969 |
| 63 | 〃 | 〃 | 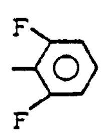 | 1 | m.p. 66～68℃ |
| 64 | 〃 | 〃 | 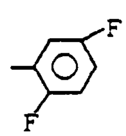 | 1 | $n_D^{26.9}$ 1.5069 |

| No. | $R^1$ | $R^2$ | $R^3$ | n | Physical property |
|---|---|---|---|---|---|
| 65 | 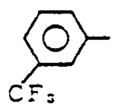 | Et | 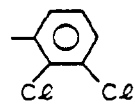 | 1 | $n_D^{19.9}$ 1.5562 |
| 66 | 〃 | 〃 | 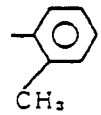 | 1 | m.p. 76~77℃ |
| 67 | 〃 | 〃 | 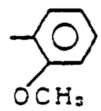 | 1 | |
| 68 | 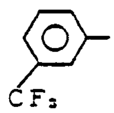 | 〃 | 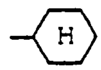 | 1 | m.p. 53~55℃ |
| 69 | 〃 | 〃 | 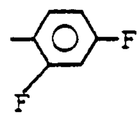 | 0 | $n_D^{26.4}$ 1.5164 |
| 70 | 〃 | 〃 | 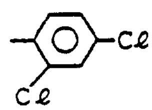 | 0 | $n_D^{27.2}$ 1.5419 |
| 71 | 〃 | 〃 | 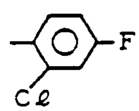 | 0 | $n_D^{27.2}$ 1.5286 |
| 72 | 〃 | 〃 | 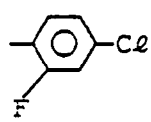 | 0 | $n_D^{27.2}$ 1.5207 |
| 73 | 〃 | 〃 | 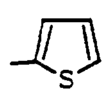 | 1 | m.p. 28~30℃ |
| 74 | 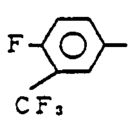 | 〃 | 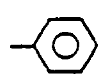 | 1 | $n_D^{23.0}$ 1.5207 |
| 75 | 〃 | 〃 | 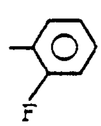 | 1 | m.p. 74~76℃ |

| No. | R[1] | R[2] | R[3] | n | Physical property |
|---|---|---|---|---|---|
| 76 | 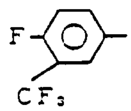 | Et | 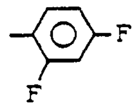 | 0 | $n_D^{26.8}$ 1.5110 |
| 77 | 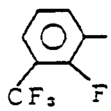 | 〃 | 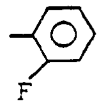 | 1 | $n_D^{26.8}$ 1.5164 |
| 78 | 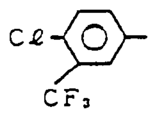 | 〃 | 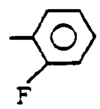 | 1 | m.p. 127～129℃ |
| 79 | 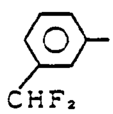 | 〃 | 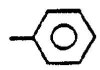 | 1 | |
| 80 | 〃 | 〃 | 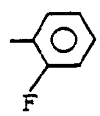 | 1 | $n_D^{26.4}$ 1.5375 |
| 81 | 〃 | 〃 | 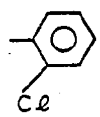 | 1 | $n_D^{27.0}$ 1.5524 |
| 82 | 〃 | 〃 | 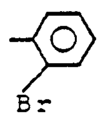 | 1 | |
| 83 | 〃 | 〃 | 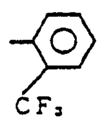 | 1 | |
| 84 | 〃 | 〃 | 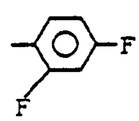 | 0 | |
| 85 | 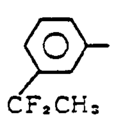 | 〃 | 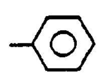 | 1 | |

| No. | $R^1$ | $R^2$ | $R^3$ | n | Physical property |
|---|---|---|---|---|---|
| 86 | 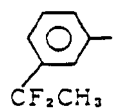 | Et | 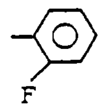 | 1 | |
| 87 | 〃 | 〃 | 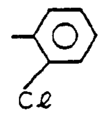 | 1 | |
| 88 | 〃 | 〃 | 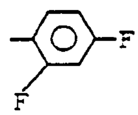 | 0 | |
| 89 | 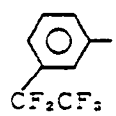 | 〃 | 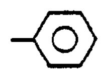 | 1 | |
| 90 | 〃 | 〃 | 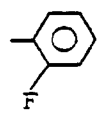 | 1 | |
| 91 | 〃 | 〃 | 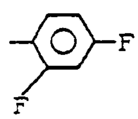 | 1 | |
| 92 | 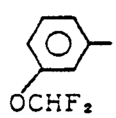 | 〃 | 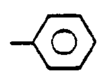 | 1 | $n_D^{23.0}$ 1.5414 |
| 93 | 〃 | 〃 | 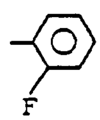 | 1 | m.p. 54～55 ℃ |
| 94 | 〃 | 〃 | 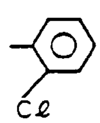 | 1 | $n_D^{25.0}$ 1.5490 |
| 95 | 〃 | 〃 | 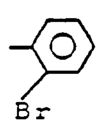 | 1 | $n_D^{26.2}$ 1.5591 |

| No. | R¹ | R² | R³ | n | Physical property |
|---|---|---|---|---|---|
| 96 | 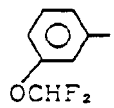 | Et | 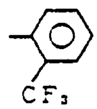 | 1 | |
| 97 | 〃 | 〃 | 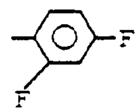 | 0 | $n_D^{27.0}$ 1.5302 |
| 98 | 〃 | 〃 | 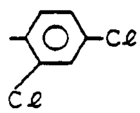 | 0 | |
| 99 | 〃 | 〃 | 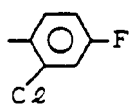 | 0 | |
| 100 | 〃 | 〃 | 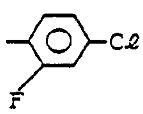 | 0 | $n_D^{24.7}$ 1.5491 |
| 101 | 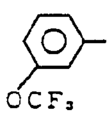 | 〃 | 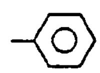 | 1 | m.p. 83～85 ℃ |
| 102 | 〃 | 〃 | 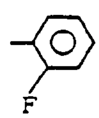 | 1 | m.p. 63～64 ℃ |
| 103 | 〃 | 〃 | 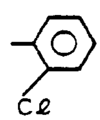 | 1 | m.p. 84～85 ℃ |
| 104 | 〃 | 〃 | 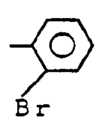 | 1 | $n_D^{26.2}$ 1.5377 |
| 105 | 〃 | 〃 | 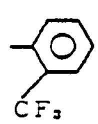 | 1 | $n_D^{26.2}$ 1.4970 |

| No. | $R^1$ | $R^2$ | $R^3$ | n | Physical property |
|---|---|---|---|---|---|
| 106 | 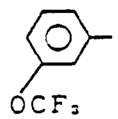 | Et | 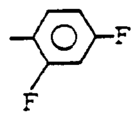 | 0 | $n_D^{27.0}$ 1.5108 |
| 107 | 〃 | 〃 | 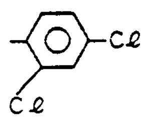 | 0 | $n_D^{27.4}$ 1.5413 |
| 108 | 〃 | 〃 | 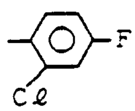 | 0 | m.p. 74～75℃ |
| 109 | 〃 | 〃 | 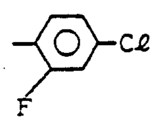 | 0 | m.p. 69～70℃ |
| 110 | 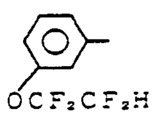 | 〃 | 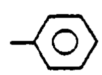 | 1 | |
| 111 | 〃 | 〃 | 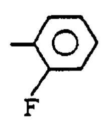 | 1 | |
| 112 | 〃 | 〃 | 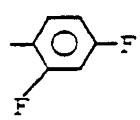 | 0 | |
| 113 | 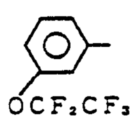 | 〃 | 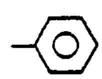 | 1 | |
| 114 | 〃 | 〃 | 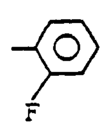 | 1 | |
| 115 | 〃 | 〃 | 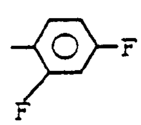 | 0 | |

| No. | $R^1$ | $R^2$ | $R^3$ | n | Physical property |
|---|---|---|---|---|---|
| 116 | 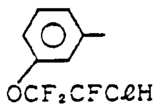 | Et | 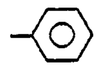 | 1 | |
| 117 | 〃 | 〃 | 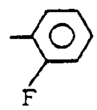 | 1 | |
| 118 | 〃 | 〃 | 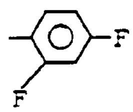 | 0 | |
| 119 | 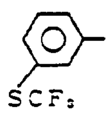 | 〃 | 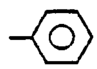 | 1 | |
| 120 | 〃 | 〃 | 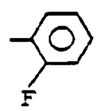 | 1 | m.p. 82～83℃ |
| 121 | 〃 | 〃 | 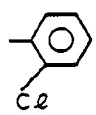 | 1 | m.p. 96～98℃ |
| 122 | 〃 | 〃 | 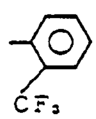 | 1 | m.p. 73～75℃ |
| 123 | 〃 | 〃 | 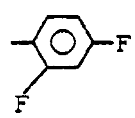 | 0 | m.p. 70～72℃ |
| 124 | 〃 | 〃 | 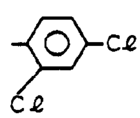 | 0 | |
| 125 | 〃 | 〃 | 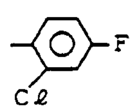 | 0 | |

| No. | $R^1$ | $R^2$ | $R^3$ | n | Physical property |
|---|---|---|---|---|---|
| 126 | 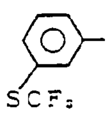 | Et | 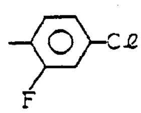 | 0 | |
| 127 | 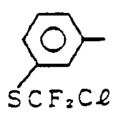 | 〃 | 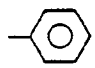 | 1 | |
| 128 | 〃 | 〃 | 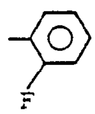 | 1 | |
| 129 | 〃 | 〃 | 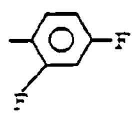 | 0 | |
| 130 | 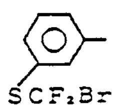 | 〃 | 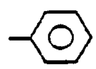 | 1 | |
| 131 | 〃 | 〃 | 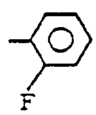 | 1 | |
| 132 | 〃 | 〃 | 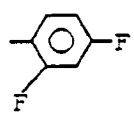 | 0 | |
| 133 | 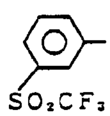 | 〃 | 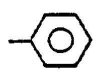 | 1 | |
| 134 | 〃 | 〃 | 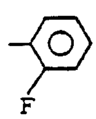 | 1 | |
| 135 | 〃 | 〃 | 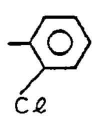 | 1 | |

| No. | $R^1$ | $R^2$ | $R^3$ | n | Physical property |
|---|---|---|---|---|---|
| 136 | 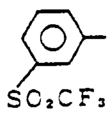 | Et | 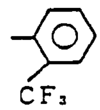 | 1 | |
| 137 | 〃 | 〃 | 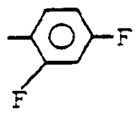 | 0 | |
| 138 | 〃 | 〃 | 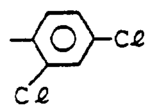 | 0 | |
| 139 | 〃 | 〃 | 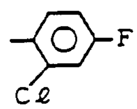 | 0 | |
| 140 | 〃 | 〃 | 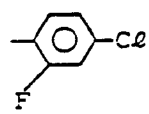 | 0 | |
| 141 | 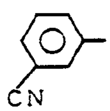 | 〃 | 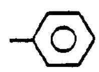 | 1 | m.p. 137~139℃ |
| 142 | 〃 | 〃 | 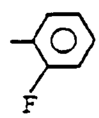 | 1 | m.p. 123~124℃ |
| 143 | 〃 | 〃 | 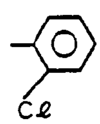 | 1 | m.p. 131~132℃ |
| 144 | 〃 | 〃 | 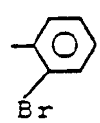 | 1 | |
| 145 | 〃 | 〃 | 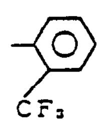 | 1 | m.p. 111~113℃ |

| No. | $R^1$ | $R^2$ | $R^3$ | n | Physical property |
|---|---|---|---|---|---|
| 146 | 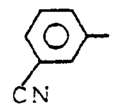 | Et | 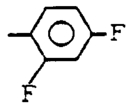 | 0 | m.p. 94～95℃ |
| 147 | ″ | ″ | 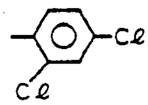 | 0 | m.p. 65～67℃ |
| 148 | ″ | ″ | 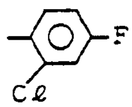 | 0 | |
| 149 | ″ | ″ | 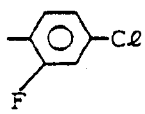 | 0 | m.p. 79～81℃ |
| 150 | 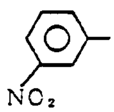 | ″ | 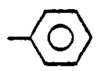 | 0 | m.p. 107～108℃ |
| 151 | ″ | ″ | 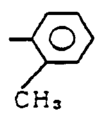 | 1 | m.p. 99～101℃ |
| 152 | ″ | ″ | 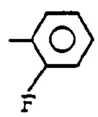 | 1 | m.p. 100～101℃ |
| 153 | ″ | ″ | 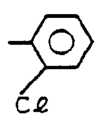 | 1 | m.p. 105～107℃ |
| 154 | ″ | ″ | 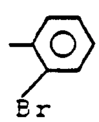 | 1 | |
| 155 | ″ | ″ | 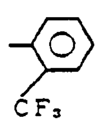 | 1 | m.p. 104～106℃ |
| 156 | ″ | ″ | 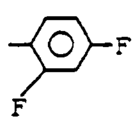 | 0 | m.p. 87～88℃ |

| No. | R¹ | R² | R³ | n | Physical property |
|---|---|---|---|---|---|
| 157 | 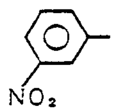 | Et | 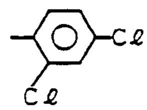 | 0 | m.p. 93～94℃ |
| 158 | 〃 | 〃 | 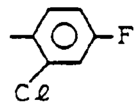 | 0 | |
| 159 | 〃 | 〃 | 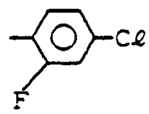 | 0 | m.p. 87～89℃ |
| 160 | 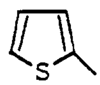 | 〃 | 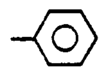 | 1 | m.p. 125～126℃ |
| 161 | 〃 | 〃 | 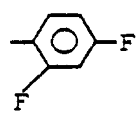 | 0 | m.p. 53～55℃ |
| 162 | 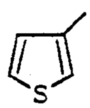 | 〃 | 〃 | 0 | m.p. 71～72℃ |
| 163 | 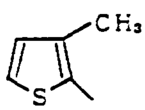 | 〃 | 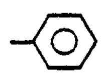 | 1 | $n_D^{27.0}$ 1.5867 |
| 164 | 〃 | 〃 | 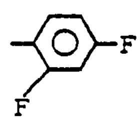 | 0 | $n_D^{27.0}$ 1.5539 |
| 165 | 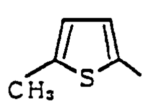 | 〃 | 〃 | 0 | $n_D^{25.8}$ 1.5645 |
| 166 | 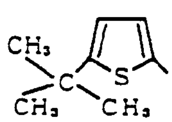 | 〃 | 〃 | 0 | $n_D^{24.0}$ 1.5468 |

| No. | $R^1$ | $R^2$ | $R^3$ | n | Physical property |
|---|---|---|---|---|---|
| 167 | 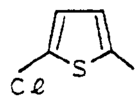 | Et | 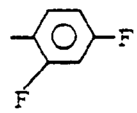 | 0 | $n_D^{25.6}$ 1.5682 |
| 168 | 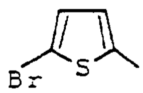 | 〃 | 〃 | 0 | $n_D^{27.8}$ 1.5796 |
| 169 | 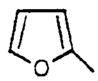 | 〃 | 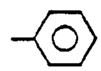 | 1 | m.p. 116~118℃ |
| 170 | 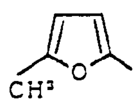 | 〃 | 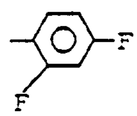 | 1 | $n_D^{28.0}$ 1.5456 |

Provided that Me and Et each represent $-CH_3$ and $-C_2H_5$, respectively.

Also, in the present invention, the benzylideneaminoxyalkanoic acid thioamide derivative (Q = S) is represented by the following formula:

$$R^1-CH=N-O-\underset{R^2}{\underset{|}{CH}}-\overset{S}{\overset{\|}{C}}-NH-(CH_2)_n-R^3 \qquad (I'')$$

wherein $R^1$, $R^2$, $R^3$ and n have the same meanings as defined above.

In the above formula, $R^1$ may include a substituted or unsubstituted phenyl group and a substituted or unsubstituted thienyl group, but preferably a substituted or unsubstituted phenyl group. As the substituent for the phenyl group, there may be mentioned a halogen atom (e.g. a chlorine atom, a bromine atom, a fluorine atom and an iodine atom), a halo-lower alkyl group (e.g. a trifluoromethyl group, a difluoromethyl group, a 1,1-difluoroethyl group, a 1,1,2,2-tetrafluoroethyl group and a pentafluoromethyl group), a halo-lower alkoxy group (e.g. a difluoromethoxy group, a trifluoromethoxy group and a 1,1,2-trifluoro-2-chloroethoxy group), a cyano group, a nitro group, a lower alkyl group, a lower alkoxy group, a halo-lower alkylthio group and a halo-lower alkylsulfonyl group, but preferably a halogen atom, a halo-lower alkyl group, a halo-lower alkoxy group, a cyano group and a nitro group, and more preferably a fluorine atom as the halogen atom, a trifluoromethyl group as the halo-lower alkyl group, and a difluoromethoxyl group and a trifluoromethoxyl group as the halo-lower alkoxy group. Further preferably, a position of each substituent is 3-position.

$R^2$ may be mentioned a straight or branched alkyl group having 1 to 6 carbon atoms, but preferably an alkyl group having 1 to 4 carbon atoms, and more preferably an ethyl group.

$R^3$ may be a phenyl group, which may be substituted by a chlorine or fluorine atom, or a substituted or unsubstituted thienyl group. Further preferably, a position(s) of each substituent is/are 2-position and/or 4-position.

n represents 0 or 1.

As the novel benzylideneaminoxyalkanoic acid thioamide derivative (I'') which is the title compound, there may be mentioned an optical isomer based on an asymmetric carbon atom.

The compound of the formula (I') can be prepared by either one of the methods A to D shown below.

Method A

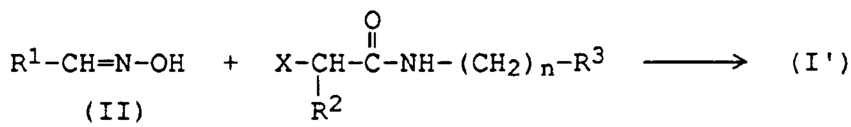

(II) (III) (I')

wherein $R^1$, $R^2$, $R^3$, n and X have the same meanings as defined above.

The above method can be carried out by reacting an oxime compound (II) and 2-halogenoalkanoic acid amide (III) in a suitable solvent in the presence of a base. The solvent is not particularly limited so long as it does not participate the reaction, and there may be mentioned a ketone such as acetone, methyl ethyl ketone and methyl isobutyl ketone; a bipolar aprotic solvent such as dimethylformamide and dimethylsulfoxide; and an ether such as tetrahydrofuran. As the base, potassium carbonate, sodium carbonate and the like may be used. The reaction is substantially completed by stirring at 20 to 150 °C for one to 10 hours.

Method B

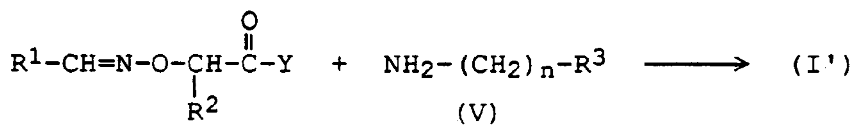

(IV) (V) (I')

wherein $R^1$, $R^2$, $R^3$, n and Y have the same meanings as defined above.

The above method can be carried out by reacting an acid halide (IV) and an amine (V) in a suitable solvent in the presence of a base. The solvent is not particularly limited so long as it does not participate the reaction, and there may be mentioned an aromatic hydrocarbon such as benzene, toluene and xylene; an ether such as diethyl ether, 1,4-dioxane and tetrahydrofuran; an aliphatic hydrocarbon such as n-hexane; and a halogenated hydrocarbon such as chloroform and methylene chloride. As the base, while the amine (V) acts such a role but pyridine, triethylamine and the like may be used. The reaction is substantially completed by stirring at 0 to 50 °C for one to 5 hours.

Method C

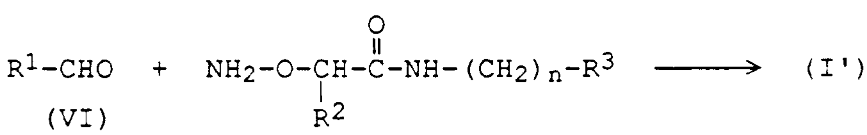

(VI) (VII) (I')

wherein $R^1$, $R^2$, $R^3$ and n have the same meanings as defined above.

The above method can be carried out by reacting an aldehyde (VI) and an aminoxyalkanoic acid amide (VII) in the absence or presence of a solvent. The solvent is not particularly limited so long as it does not participate the reaction, and there may be mentioned an alcohol such as methanol and ethanol; an ether such as diethyl ether, tetrahydrofuran and 1,4-dioxane; an aliphatic hydrocarbon such as n-hexane; and an aromatic hydrocarbon such as benzene, toluene and xylene. The reaction is substantially completed by stirring at 0 to 50 °C for one to 5 hours.

Method D

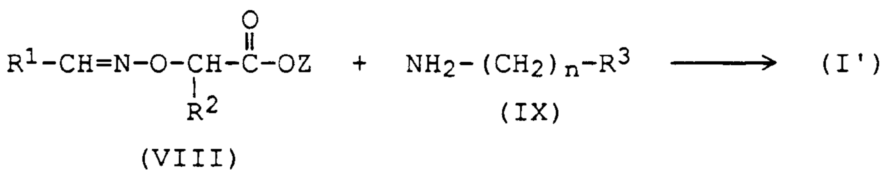

(VIII) (IX) (I')

wherein $R^1$, $R^2$, $R^3$, n and Z have the same meanings as defined above.

The above method can be carried out by reacting an ester (VIII) and an amine (IX) in a suitable solvent in the presence of a base. The solvent is not particularly limited so long as it does not participate the reaction, and there may be mentioned an aromatic hydrocarbon such as benzene, toluene and xylene; and an alcohol such as methanol and ethanol. As the base, an alkali metal salt of an alcohol such as sodium methoxide and the like may be used. The reaction is substantially completed by stirring at 10 to 100 °C for one to 20 hours.

Also, the title compound (I″) of the present invention can be prepared by the following method.

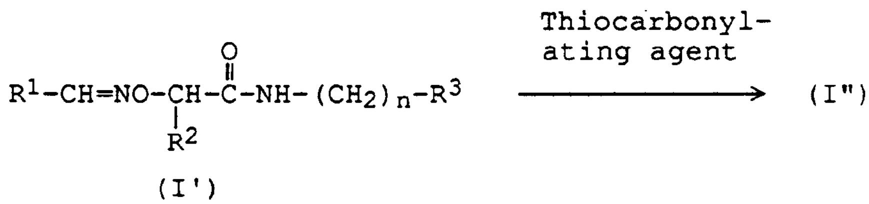


(I') (I")

wherein $R^1$, $R^2$, $R^3$ and n have the same meanings as defined above.

Preferred compounds included in the formula (I″) are enumerated in Table 2.

Table 2

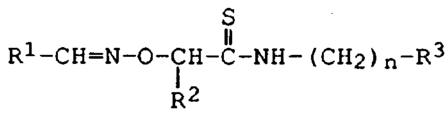

| Compound | $R^1$ | $R^2$ | $R^3$ | n | Physical property |
|---|---|---|---|---|---|
| 171 | 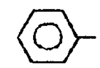 | $C_2H_5$ | 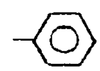 | 0 | $n_D^{27.2}$ 1.5945 |
| 172 | 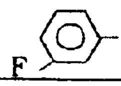 | 〃 | 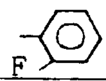 | 1 | $n_D^{22.7}$ 1.5844 |
| 173 | 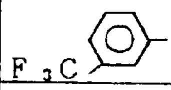 | 〃 | 〃 | 1 | $n_D^{22.8}$ 1.5416 |
| 174 | 〃 | 〃 | 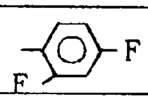 | 0 | $n_D^{25.8}$ 1.5366 |
| 175 | 〃 | 〃 | 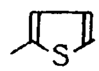 | 1 | $n_D^{22.4}$ 1.5748 |
| 176 | 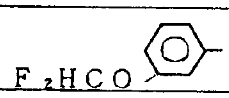 | 〃 | 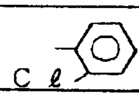 | 1 | $n_D^{22.4}$ 1.5860 |
| 177 | 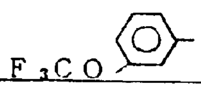 | 〃 | 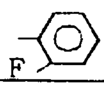 | 1 | $n_D^{22.4}$ 1.5506 |
| 178 | 〃 | 〃 | 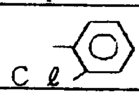 | 1 | $n_D^{22.4}$ 1.5660 |
| 179 | 〃 | 〃 | 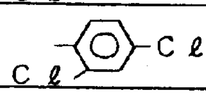 | 0 | m.p.57~59℃ |
| 180 | 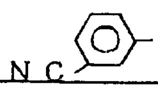 | 〃 | 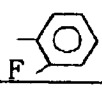 | 1 | Viscous liquid |
| 181 | 〃 | 〃 | 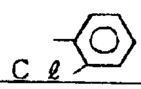 | 1 | m.p.54~59℃ |
| 182 | 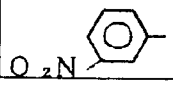 | 〃 | 〃 | 1 | $n_D^{22.4}$ 1.6170 |

The above compound (I") can be prepared by reacting the starting compound (I') and a thiocarbonylating agent in a solvent. As the thiocarbonylating agent, there may be mentioned phosphorus sulfide such as phosphorus pentasulfide and Lawesson's reagent, but preferably phosphorus pentasulfide.

Preparation of the above compound (I") can be carried out with a reaction concentration of 5 to 60 % by weight. A ratio of the starting compound (I') and the thiocarbonylating agent is 1 to 3 moles of the thiocarbonylating agent, preferably 1.2 to 2.0 moles per mole of the starting compound (I').

A reaction temperature is not particularly limited so long as the reaction is carried out below the boiling

temperature of the solvent to be used, but generally carried out at room temperature or more, preferably 20 to 140 °C, and it is further preferred to shorten the reaction time by heating.

A reaction time may vary depending on the above concentration and the temperature but generally carried out for 0.5 to 12 hours.

The title compounds (I) of the present invention show excellent herbicidal effect with high selectivity, i.e. they show excellent herbicidal effect against not only grass weeds (e.g. barnyard grass, annual poa and mannagrass) and general broadleaved weeds (e.g. common lambsquaters, common purslane, curly dock, livid amaranthus and chickweed) but also hardly removable broadleaved weeds (e.g. viola, cleavers, speedwell and cocklebur) and further have no chemical damage against young seedling paddy rice plant, wheat, soybean, corn and the like

The herbicide of the present invention contains at least one compound (I) as an effective ingredient.

The compound (I) may be used alone but it is preferred to use it by formulating a carrier, surfactant, dispersing agent, auxiliary and the like (e.g. prepared as a composition such as dustablepowder, emulsifiable concentrate, fine granule, granule, wettable powder, suspension concentrate and aerosol) according to the conventional method.

As the carrier, there may be mentioned, for example, a solid carrier such as talc, bentonite, clay, kaolin, mica, diatomaceous earth, white carbon, vermiculite, dolomite, zeolite, gypsum, calcium carbonate, magnesium lime, phosphor lime, slaked lime, quartz sand, anhydrous silicic acid, synthesized calcium silicate, ammonium sulfate, urea, wood powder, starch and cellulose; a liquid carrier such as hydrocarbons (kerosine and mineral oil), aromatic hydrocarbons (benzene, toluene, xylene, ethylbenzene, cumene and methylnaphthalene), halogenated hydrocarbons (chloroform and carbon tetrachloride), ethers (dioxane and tetrahydrofuran), ketones (acetone, cyclohexanone and isophorone), esters (ethyl acetate, ethylene glycol acetate, ethylene glycol phenyl ether and dibutyl maleate), alcohols (methanol, n-hexanol, cyclohexanol and ethylene glycol), polar solvents (dimethylformamide and dimethylsulfoxide) and water; and a gaseous carrier such as air, nitrogen, carbon dioxide gas and fleon (in this case, mixed jetting can be carried out).

As a surfactant which can be used for improving attachment and absorption of the present herbicide to plant or improving characteristics such as dispersing, emulsifying, wetting, spreading and stabilizing chemicals, there may be mentioned a nonionic, anionic, cationic and amphoteric surfactants such as alcohol sulfates, alkylsulfonates, lignine sulfonates and polyoxyethylene glycol ethers. Also, for improving characteristics of the preparations, carboxymethylcellulose, polyoxyethylene glycol and gum arabic may be used as auxiliaries.

In the preparation of the present herbicide, in addition to the above carrier, surfactant, dispersing agent and auxiliary, other pesticide (fungicide and insecticide), fertilizer (urea, ammonium sulfate, ammonium phosphate and potassium salt) and soil conditioner may be optionally formulated depending on the purpose each independently or in combination. Also, it may be optionally used in combination of the other herbicides.

An effective concentration of the component when the compound (I) of the present invention is making into preparation is generally 1 to 50 % by weight in emulsifiable concentrate, 0.3 to 25 % by weight in dustable powder, 1 to 90 % by weight in wettable powder, 0.5 to 5 % by weight in granule, 2 to 40 % by weight in suspension concentrate and 0.1 to 5 % by weight in aerosol.

These preparations can be applied to each use by spreading them to surface of plant stalk and leave, soil or paddy field depending on the purpose after diluting them to a suitable concentration or directly applied. The herbicide of the present invention can be expected to have selective effect without chemical damage to paddy rice plant, soybean, corn and wheat as a soil treatment agent before germination of a paddy field or a upland field and also effective as a foliar treatment agent. A dose to be used may be 1 to 1000 g per 10 are.

In the following, the present invention is explained by referring to Examples, but the present invention is not limited by these Examples.

Example 1 (Synthesis method A)

Synthesis of N-benzyl-2-(3-chlorobenzylideneaminoxy)butanoic acid amide (Compound No. 40)

In 30 ml of acetone were dissolved 1.7 g (0.011 mole) of m-chlorobenzaldoxime and 2.6 g (0.01 mole) of N-benzyl-2-bromobutanoic acid amide, and 1.51 g (0.011 mole) of anhydrous potassium carbonate was added to the solution and the mixture was refluxed for 4 hours under heating. After completion of the reaction, the reaction mixture was added to water, and separated oily product was extracted with ethyl acetate. After ethyl acetate was washed with water and dried over magnesium sulfate, the residue obtained by evaporation of ethyl acetate under reduced pressure was purified by column chromatography (Wako gel C-200 (trade name, produced by Wako Junyaku K.K., eluted by toluene : ethyl acetate = 4 : 1) to obtain 2.3 g (Yield: 69 %) of the title compound having a melting point of 75 to 78 °C.

Example 2 (Synthesis method B)

Synthesis of N-(2,5-difluorobenzyl)-2-(3-trifluoromethylbenzylideneaminoxy)butanoic acid amide (Compound No. 64)

To 20 ml of toluene were added 2.9 g (0.02 mole) of 2,5-difluorobenzylamine and 2.2 g (0.22 mole) of triethylamine and after cooling to 5 °C, 6.0 g (0.02 mole) of 3-trifluoromethylbenzylideneaminoxybutanoic acid chloride was added dropwise. After completion of the dropwise addition, the mixture was stirred at room temperature for 2 hours. Subsequently, the reaction mixture was washed with water, the toluene layer was dried over anhydrous sodium sulfate and the residue obtained by removing toluene under reduced pressure was treated in the same manner as in Example 1 to obtain 7.0 g (Yield: 89 %) of the title compound having a refractive index of $n_D^{26.9}$ = 1.5069.

Example 3 (Synthesis method C)

Synthesis of N-(2,4-difluorophenyl)-2-(3-nitrobenzylideneaminoxy)butanoic acid amide (Compound No. 156)

In 20 ml of ethanol were dissolved 1.51 g (0.01 mole) of m-nitrobenzaldehyde and 2.3 g (0.01 mole) of N-(2,4-difluorophenyl)-2-aminoxybutanoic acid amide, and the mixture was reacted at 60 °C for 2 hours. After completion of the reaction, ethanol was removed under reduced pressure. The residue obtained was treated in the same manner as in Example 1 to obtain 3.0 g (Yield: 83 %) of the title compound as white powder having a melting point of 87 to 88 °C.

Example 4 (Synthesis method D)

Synthesis of N-benzyl-2-(3-trifluoromethylbenzylideneaminoxy)butanoic acid amide (Compound No. 58)

After dissolving 3.0 g (0.01 mole) of ethyl 2-(3-trifluoromethylbenzylideneaminoxy)butanoate and 1.1 g (0.01 mole) of benzylamine in 15 ml of methanol, 1.0 g (0.005 mole) of sodium methoxide (28 % methanol solution) was added to the solution and the mixture was stirred at 50 °C for 5 hours. After completion of stirring, water was added thereto and separated oily product was extracted with ethyl acetate. The ethyl acetate layer was washed with water and dried over magnesium sulfate, and evaporated under reduced pressure. The residue obtained was treated in the same manner as in Example 1 to obtain 2.9 g (Yield: 79 %) of the title compound having a refractive index of $n_D^{26.5}$ = 1.5321.

Example 5

Synthesis of [N-(2-fluorobenzyl)-2-(3-trifluoromethylbenzylideneaminoxy)butanoic acid thioamide (Compound No. 173)

In xylene (250 ml) was dissolved N-(2-fluorobenzyl)-2-(3-trifluoromethylbenzylideneaminoxy)butanoic acid amide (10.0 g, 26.1 mmole), and then phosphorus pentasulfide (13.4 g, 30.1 mmole) was added to the solution and the mixture was stirred while maintaining to 70 to 80 °C for about 6 hours under heating.

After completion of the reaction, insolubles were removed, then the solvent was removed under reduced pressure and the residue obtained was isolated by silica gel column chromatography (Wako gel C-200, trade name, produced by Wako Junyaku K.K., eluted by hexane : ethyl acetate = (19 : 1) to (7 : 3)) to obtain 6.55 g of the title compound (I″) (Compound No. 173 shown in Table 2).

Example 6

Synthesis of [N-(2-chlorobenzyl)-2-(3-trifluoromethoxybenzylideneaminoxy)butanoic acid thioamide (Compound No. 178)

In THF (200 ml) was dissolved N-(2-chlorobenzyl)-2-(3-trifluoromethoxybenzylideneaminoxy)butanoic acid amide (4.2 g, 10.1 mmole), and Lawesson's reagent(5.0 g, 12.3 mmole) was added to the solution and the mixture was stirred while maintaining to 40 °C for about one hour under heating.

After completion of the reaction, insolubles were removed, then the solvent was removed under reduced pressure and the residue obtained was treated in the same manner as in Example 5 to obtain 3.9 g of the title compound (I″) (Compound No. 178 shown in Table 2).

Example 7

Synthesis of N-(2-chlorobenzyl)-2-(3-cyanobenzylideneaminoxy)butanoic acid thioamide (Compound No. 181)

In 1,2-dichloroethane (100 ml) was dissolved N-(2-chlorobenzyl)-2-(3-cyanobenzylideneaminoxy)butanoic acid amide (4.98 g, 14.0 mmole), and then phosphorus pentasulfide (8.10 g, 18.2 mmole) was added to the solution and the mixture was stirred while maintaining to 50 to 60 °C for about one hour under heating.

After completion of the reaction, insolubles were removed, then the solvent was removed under reduced pressure and the residue obtained was treated in the same manner as in Example 5 to obtain 4.85 g of the title compound (I″) (Compound No. 181 shown in Table 2).

Example 8

Synthesis of Compounds No.s 171, 172, 174, 175 to 177, 179, 180 and 182

In the same synthetic method as in the above (1) to (3), the title compounds (I″) (shown in Table 2 as Compounds No.s 171, 172, 174, 175 to 177, 179, 180 and 182) can be obtained by using starting materials (I′) having substituents ($R^1$, $R^2$, $R^3$ and n) shown in Table 2 and a thiocarbonylating agent.

Example 9 Preparation of granules

After 8 parts by weight of N-benzyl-2-(3-trifluoromethylbenzylideneamonoxy)butanoic acid amide (compound of Compound No. 58), 30 parts by weight of bentonite, 59 parts by weight of talc, 1 part by weight of Neopelex powder (trade name, produced by Kao Atlas K.K.) and 2 parts by weight of sodium lignosulfate were kneaded, small amount of water was added thereto and the mixture was kneaded again, the kneaded product was granulated and dried to obtain granules.

Example 10 Preparation of wettable powder

N-(2-chlorobenzyl)-2-(3-trifluoromethylbenzylideneaminoxy)butanoic acid amide (compound of Compound No. 60) (50 parts by weight), 48 parts by weight of kaolin and 2 parts by weight of Neopelex powder (trade name) were uniformly mixed and pulverized to obtain a fine powdery wettable powder.

Example 11 Preparation of emulsifiable concentrate

N-(2-chlorobenzyl)-2-(3-methylbenzylideneaminoxy)butanoic acid amide (compound of Conpound No. 13) (30 parts by weight), 60 parts by weight of xylene, 5 parts by weight of dimethylformamide and 5 parts by weight of Toxanone (trade name, produced by Sanyo Kasei Kogyo K.K.) were mixed and dissolved to obtain an emulsifiable concentrate.

Example 12 Preparation of dustable powder

N-benzyl-2-(3-trifluoromethylbenzylideneaminoxy)butanoic acid amide (compound of Compound No. 60) (5 parts by weight), 50 parts by weight of talc and 45 parts by weight of kaolin were uniformly mixed to obtain dustable powder.

Example 13 (Soil treatment test for paddy field weed)

Wagner pots, each having an area of 1/5000 are, were packed with Ube soil (alluvial soil) and planted with uniformly seeds or tubers of barnyardgrass, monochoria, slender spikerush and bulrush, and rice plant seedlings (young seedling paddy rice plant) of 1.8 to 2 leaf stage were also transplanted. Then, the pots were filled with water to a depth of 3 cm.

Each wettable powder containing the title compound (I) prepared in accordance with Example 10 was subjected to dropwise addition treatment by using pipet after diluting with water containing a surfactant (0.05 %) with an effective amount of 20 g/are at 1 leaf stage of barnyardgrass. These plants were controlled in a green house at an average temperature of 25 °C for 3 weeks. and then herbicidal effects thereof were investigated.

The results are shown in Table 3 wherein the herbicidal effects are evaluated according to the rating system as defined below as compared with non-treated district.

5: All killed, 4: Severely damaged, 3: Moderately damaged, 2: Slightly damaged, 1: Minor damaged, and 0: None (normal development)

Table 3

| Compound No. | Seedling rice plant | Barnyard-grass | Mono-choria | Slender spikerush | Bulrush |
|---|---|---|---|---|---|
| 10 | 0 | 5 | 5 | 5 | 5 |
| 23 | 0 | 5 | 5 | 4 | 5 |
| 26 | 1 | 5 | 5 | 5 | 5 |
| 27 | 1 | 5 | 5 | 5 | 5 |
| 28 | 1 | 5 | 5 | 5 | 5 |
| 30 | 1 | 5 | 5 | 5 | 5 |
| 40 | 1 | 5 | 5 | 5 | 5 |
| 41 | 1 | 5 | 5 | 5 | 5 |
| 42 | 1 | 5 | 5 | 5 | 5 |
| 54 | 1 | 5 | 5 | 5 | 5 |
| 55 | 1 | 5 | 5 | 5 | 5 |
| 58 | 2 | 5 | 5 | 5 | 5 |
| 59 | 2 | 5 | 5 | 5 | 5 |
| 60 | 1 | 5 | 5 | 5 | 5 |
| 61 | 0 | 4 | 5 | 4 | 5 |
| 62 | 0 | 5 | 5 | 5 | 5 |
| 63 | 1 | 5 | 5 | 5 | 5 |
| 64 | 1 | 5 | 5 | 5 | 5 |
| 68 | 1 | 5 | 5 | 5 | 5 |
| 72 | 1 | 5 | 5 | 5 | 5 |
| 73 | 1 | 5 | 5 | 5 | 5 |
| 75 | 1 | 5 | 5 | 5 | 5 |
| 80 | 1 | 5 | 5 | 5 | 5 |
| 81 | 1 | 5 | 5 | 5 | 5 |
| 93 | 1 | 5 | 5 | 5 | 5 |
| 94 | 1 | 5 | 5 | 5 | 5 |
| 101 | 0 | 5 | 5 | 5 | 5 |
| 102 | 2 | 5 | 5 | 5 | 5 |
| 103 | 2 | 5 | 5 | 5 | 5 |
| 141 | 0 | 5 | 5 | 5 | 5 |
| 142 | 1 | 5 | 5 | 5 | 5 |

Table 3 (Contd)

| Compound No. | Seedling rice plant | Barnyard-grass | Mono-choria | Slender spikerush | Bulrush |
|---|---|---|---|---|---|
| 152 | 0 | 5 | 5 | 5 | 5 |
| 151 | 0 | 5 | 5 | 5 | 5 |
| 171 | 0 | 4 | 5 | 5 | 5 |
| 172 | 0 | 5 | 5 | 5 | 5 |
| 173 | 2 | 5 | 5 | 5 | 5 |
| 174 | 1 | 5 | 5 | 5 | 5 |
| 175 | 1 | 5 | 5 | 5 | 5 |
| 176 | 0 | 5 | 5 | 5 | 5 |
| 177 | 1 | 5 | 5 | 5 | 5 |
| 178 | 1 | 5 | 5 | 5 | 5 |
| 179 | 0 | 5 | 5 | 5 | 5 |
| 180 | 0 | 4 | 5 | 5 | 5 |
| 181 | 0 | 5 | 5 | 5 | 5 |
| 182 | 0 | 5 | 5 | 5 | 5 |

Example 14 (Soil treatment test for upland weed control)

Wagner pots, each having an area of 1/4000 are, were packed with Ube soil (diluvial soil) and then each seed of corn, soybean, wheat, barnyardgrass, crabgrass, livid amaranthus, common lambsquarters, common purslane and curly dock were sowed therein. After covering with soil, each herbicide was uniformly sprayed on the surface of soil by using a pressure sprayer so as to become an effective amount of 20 g/are and these plants were controlled in a green house at an average temperature of 25 °C. Three weeks after the treatment, the herbicidal effects of each test compound were investigated. The results are shown in Table 4 wherein the herbicidal effects are evaluated according to the rating system as defined below.

5: All killed, 4: Severely damaged, 3: Moderately damaged, 2: Slightly damaged, 1: Minor damaged, and 0: None (normal development)

Table 4

| Compound No. | Soybean | Corn | Wheat | Barnyardgrass | Crabgrass | Livid amaranthus | Common lambsquarters | Common purslane | Curly dock |
|---|---|---|---|---|---|---|---|---|---|
| 3 | 0 | 0 | 0 | 3 | 5 | 5 | 5 | 5 | 4 |
| 11 | 0 | 0 | 0 | 4 | 5 | 5 | 4 | 4 | 3 |
| 26 | 0 | 0 | 0 | 4 | 5 | 5 | 5 | 5 | 4 |
| 27 | 0 | 0 | 0 | 4 | 5 | 5 | 5 | 5 | 4 |
| 30 | 1 | 1 | 1 | 5 | 5 | 5 | 5 | 5 | 4 |
| 37 | 1 | 0 | 0 | 4 | 5 | 5 | 5 | 5 | 5 |
| 41 | 0 | 0 | 1 | 5 | 5 | 5 | 5 | 5 | 5 |
| 42 | 0 | 0 | 1 | 4 | 5 | 5 | 5 | 5 | 4 |
| 54 | 0 | 1 | 0 | 4 | 5 | 5 | 5 | 4 | 5 |
| 55 | 0 | 1 | 1 | 5 | 5 | 5 | 5 | 5 | 5 |
| 57 | 0 | 0 | 2 | 5 | 5 | 5 | 5 | 5 | 5 |
| 58 | 0 | 0 | 1 | 4 | 5 | 5 | 5 | 5 | 5 |
| 59 | 1 | 2 | 1 | 5 | 5 | 5 | 5 | 5 | 5 |
| 60 | 1 | 0 | 1 | 5 | 5 | 5 | 5 | 5 | 5 |
| 61 | 0 | 0 | 0 | 4 | 5 | 5 | 5 | 5 | 4 |
| 62 | 1 | 1 | 1 | 5 | 5 | 5 | 5 | 5 | 5 |
| 63 | 1 | 1 | 1 | 4 | 5 | 5 | 5 | 4 | 5 |
| 64 | 1 | 2 | 1 | 4 | 5 | 5 | 5 | 5 | 5 |
| 65 | 0 | 1 | 1 | 4 | 5 | 5 | 5 | 5 | 5 |
| 66 | 1 | 1 | 0 | 5 | 5 | 5 | 5 | 5 | 5 |
| 72 | 0 | 1 | 0 | 5 | 5 | 5 | 5 | 5 | 5 |
| 73 | 0 | 0 | 0 | 5 | 5 | 5 | 5 | 5 | 5 |
| 75 | 1 | 1 | 1 | 5 | 5 | 5 | 5 | 5 | 5 |
| 80 | 0 | 0 | 0 | 5 | 5 | 5 | 5 | 5 | 5 |
| 81 | 1 | 1 | 1 | 4 | 5 | 5 | 5 | 5 | 5 |
| 93 | 0 | 0 | 0 | 5 | 5 | 5 | 5 | 5 | 5 |
| 94 | 0 | 0 | 0 | 5 | 5 | 5 | 5 | 5 | 5 |

Table 4 (Contd)

| Compound No. | Soybean | Corn | Wheat | Barnyardgrass | Crabgrass | Livid amaranthus | Common lambsquarters | Common purslane | Curly dock |
|---|---|---|---|---|---|---|---|---|---|
| 101 | 1 | 2 | 1 | 5 | 5 | 5 | 5 | 5 | 5 |
| 102 | 1 | 2 | 1 | 5 | 5 | 5 | 5 | 5 | 5 |
| 103 | 1 | 2 | 2 | 5 | 5 | 5 | 5 | 5 | 5 |
| 141 | 0 | 0 | 0 | 4 | 5 | 5 | 5 | 5 | 4 |
| 142 | 0 | 0 | 0 | 3 | 5 | 5 | 5 | 5 | 5 |
| 151 | 1 | 1 | 1 | 4 | 5 | 5 | 5 | 5 | 4 |
| 152 | 1 | 0 | 0 | 5 | 5 | 5 | 5 | 5 | 5 |

Example 15 (Soil treatment test for upland weed control)

Wagner pots, each having an area of 1/5000 are, were packed with Ube soil (alluvial soil) and then each seed of barnyardgrass, crabgrass, livid amaranthus, common lambsquarters, common purslane, curly dock, corn, soybean and wheat were sowed therein, and covered with soil.

A wettable powder of the title compounds (I) shown in Table 2 prepared according to Example 10 was diluted by water containing a surfactant (0.05 %) and uniformly sprayed on the surface of each soil by using a pressure sprayer so as to become an effective amount of the compound in each herbicide being 20 g/are. These plants were controlled in a green house at an average temperature of 25 °C for 3 weeks, then, the herbicidal effects of each test compound were investigated. The results are shown in Table 5 wherein the herbicidal effects are evaluated in the same manner as in Example 13.

Table 5

| Compound No. | Soybean | Corn | Wheat | Barnyardgrass | Crabgrass | Livid amaranthus | Common lambsquarters | Common purslane | Curly dock |
|---|---|---|---|---|---|---|---|---|---|
| 171 | 0 | 0 | 0 | 3 | 5 | 5 | 5 | 5 | 4 |
| 172 | 0 | 0 | 0 | 4 | 5 | 5 | 5 | 4 | 5 |
| 173 | 1 | 1 | 1 | 5 | 5 | 5 | 5 | 5 | 5 |
| 174 | 1 | 1 | 1 | 5 | 5 | 5 | 5 | 5 | 5 |
| 175 | 1 | 0 | 1 | 5 | 5 | 5 | 5 | 5 | 5 |
| 176 | 0 | 0 | 0 | 5 | 5 | 5 | 5 | 5 | 5 |
| 177 | 0 | 0 | 0 | 5 | 5 | 5 | 5 | 5 | 5 |
| 178 | 0 | 0 | 0 | 5 | 5 | 5 | 5 | 5 | 5 |
| 179 | 1 | 0 | 0 | 5 | 5 | 5 | 5 | 5 | 5 |
| 180 | 0 | 0 | 0 | 4 | 5 | 5 | 5 | 5 | 4 |
| 181 | 0 | 0 | 0 | 5 | 5 | 5 | 5 | 5 | 5 |
| 182 | 0 | 0 | 0 | 4 | 5 | 5 | 5 | 5 | 5 |

Example 16 Foliar spread test for upland weed control

Wagner pots, each having an area of 1/5000 are, were packed with volcanic ash soil and plants of crabgrass, barnyardgrass, common lambsquarters, livid amaranthus and cocklebur were grown by 2 leaf stage.

A wettable powder of each test compound was diluted to 2000 ppm with water containing 500 ppm of a spreading agent Neoestelin (trade name, produced by Kumiai Kagaku K.K.) and then it was uniformly sprayed to stalk and leaf by using a pressure sprayer and these plants were controlled in a green house at an average temperature of 25 °C.

Three weeks after the treatment, the herbicidal effects of each test compound were investigated. The results are shown in Table 6 wherein the herbicidal effects are evaluated according to the rating system as defined below.

5: All killed, 4: Severely damaged, 3: Moderately damaged, 2: Slightly damaged, 1: Minor damaged, and 0: None (normal development)

Table 6

| Compound No. | Barnyard-grass | Crab-grass | Cockle-bur | Common lambs-quarters | Livid amaranthus |
|---|---|---|---|---|---|
| 30 | 3 | 4 | 5 | 5 | 5 |
| 41 | 3 | 5 | 4 | 5 | 5 |
| 42 | 3 | 4 | 5 | 5 | 5 |
| 55 | 4 | 5 | 5 | 5 | 5 |
| 58 | 5 | 5 | 5 | 5 | 5 |
| 59 | 5 | 5 | 5 | 5 | 5 |
| 60 | 5 | 5 | 5 | 5 | 5 |
| 61 | 5 | 5 | 5 | 5 | 5 |
| 62 | 5 | 5 | 5 | 5 | 5 |
| 64 | 4 | 5 | 5 | 5 | 5 |
| 65 | 5 | 5 | 5 | 5 | 5 |
| 66 | 4 | 5 | 5 | 5 | 5 |
| 68 | 4 | 5 | 5 | 5 | 5 |
| 72 | 5 | 5 | 5 | 5 | 5 |
| 73 | 4 | 5 | 5 | 5 | 5 |
| 75 | 4 | 4 | 5 | 5 | 5 |
| 80 | 4 | 5 | 5 | 5 | 5 |
| 81 | 4 | 4 | 4 | 5 | 5 |
| 93 | 4 | 4 | 5 | 5 | 5 |
| 94 | 4 | 4 | 5 | 5 | 5 |
| 101 | 3 | 4 | 5 | 5 | 5 |
| 102 | 4 | 4 | 4 | 5 | 5 |
| 103 | 4 | 5 | 5 | 5 | 5 |
| 151 | 3 | 4 | 4 | 5 | 5 |
| 152 | 3 | 4 | 4 | 5 | 5 |

Example 17 Foliar spread test for upland weed control

Wagner pots, each having an area of 1/5000 are, were packed with volcanic ash soil and seeds of crabgrass, barnyardgrass, cocklebur, speedwell, chickweed and wheat were planted, covered by a soil and grown for 2 weeks.

A wettable powder of each test compound shown in Table 2 prepared according to Example 10 was diluted to 2000 ppm with water containing a surfactant (0.05 %) and then it was uniformly sprayed to the above plants by using a pressure sprayer. After these plants were controlled in a green house at an average temperature of 25 °C for 3 weeks, the herbicidal effects of each test compound were investigated.

The results are shown in Table 7 wherein the herbicidal effects are evaluated according to the same manner as in Example 13.

Table 7

| Compound No. | Wheat | Barnyard-grass | Crab-grass | Cockle-bur | Speed-well | Chick-weed |
|---|---|---|---|---|---|---|
| 172 | 0 | 3 | 4 | 4 | 4 | 4 |
| 173 | 1 | 5 | 5 | 5 | 5 | 5 |
| 177 | 0 | 5 | 5 | 5 | 5 | 5 |
| 178 | 1 | 5 | 5 | 5 | 5 | 5 |
| 181 | 0 | 4 | 4 | 4 | 5 | 3 |
| 182 | 0 | 4 | 5 | 4 | 4 | 4 |

The compounds of the present invention are, as shown in the above Table 3, when used as a soil treatment herbicide before germination of paddy field, show herbicidal effect against various weeds without any chemical damage to seedling rice plant, and as shown in Table 4, in a soil treatment before germination of upland, show good selectivity and excellent herbicidal effect against grasses and broad-leaved weeds without any chemical damage to soybean, corn and wheat. Also, as shown in Table 6, they showed excellent herbicidal effect against various weeds in foliar spread test for upland weed control.

Further, the novel benzylideneaminoxyalkanoic acid thioamide derivatives (I") of the present invention show excellent herbicidal effects against grass weeds, general broadleaved weeds and hardly removable broadleaved weeds as shown in Tables 3, 5 and 7 with high selectivity that they show no chemical damage to seedling rice plant, wheat, soybean and corn.

## Claims

### Claims for the following Contracting States : DE, FR, GB, IT

1. A benzylideneaminoxyalkanoic acid (thio)amide derivative represented by the following formula:

$$R^1\text{-CH=N-O-}\underset{\substack{|\\ R^2}}{\text{CH}}\text{-}\overset{\substack{Q\\ \|}}{\text{C}}\text{-NH-}(CH_2)_n\text{-}R^3 \qquad (I)$$

wherein Q represents an oxygen atom or a sulfur atom, n is an integer of 0 or 1, and when Q is an oxygen atom, $R^1$ represents a phenyl group which may have one or two substituents selected from the group consisting of a lower alkyl group, a lower alkoxy group, a halogen atom, a halo-lower alkyl group, a halo-lower alkoxy group, a halo-lower alkylthio group, a halo-lower alkylsulfonyl group, a cyano group and a nitro group, or a thienyl group or a furyl group which may have a substituent selected from a lower alkyl group or a halogen atom; $R^2$ represents a lower alkyl group; and $R^3$ represents a phenyl group which

may have one or two substituents selected from the group consisting of a lower alkyl group, a halogen atom, a halo-lower alkyl group and a lower alkoxy group, or a cycloalkyl group, a thienyl group or a furyl group; and when Q represents a sulfur atom, $R^1$ represents a phenyl group which may be substituted by a halogen atom, a halo-lower alkyl group, a halo-lower alkoxy group, a cyano group or a nitro group; $R^2$ represents a lower alkyl group and $R^3$ represents a phenyl group which may be substituted by a halogen atom or a thienyl group.

2. The benzylideneaminoxyalkanoic acid amide derivative according to Claim 1, wherein said derivative is represented by the formula:

$$R^1-CH=N-O-\underset{\underset{R^2}{|}}{CH}-\overset{\overset{O}{\|}}{C}-NH-(CH_2)_n-R^3 \qquad (I')$$

wherein $R^1$, $R^2$, $R^3$ and n have the same meanings as defined in Claim 1.

3. The benzylideneaminoxyalkanoic acid amide derivative according to Claim 2, wherein said lower alkyl group as the substituent(s) for $R^1$ or $R^3$ is selected from the group consisting of a methyl group, an ethyl group, a propyl group, an isopropyl group, a butyl group and a t-butyl group.

4. The benzylideneaminoxyalkanoic acid amide derivative according to Claim 2, wherein said lower alkoxy group as the substituent(s) for $R^1$ or $R^3$ is selected from the group consisting of a methoxy group and an ethoxy group.

5. The benzylideneaminoxyalkanoic acid amide derivative according to Claim 2, wherein said halogen atom as the substituent(s) for $R^1$ or $R^3$ is selected from the group consisting of a chlorine atom, a bromine atom and a fluorine atom.

6. The benzylideneaminoxyalkanoic acid amide derivative according to Claim 2, wherein said halo-lower alkyl group as the substituent(s) for $R^1$ or $R^3$ is selected from the group consisting of a trifluoromethyl group, a difluoromethyl group, a 1,1-difluoroethyl group, a 1,1,2,2-tetrafluoroethyl group and a pentafluoroethyl group.

7. The benzylideneaminoxyalkanoic acid amide derivative according to Claim 2, wherein said halo-lower alkoxy group as the substituent(s) for $R^1$ or $R^3$ is selected from the group consisting of a difluoromethoxy group, a trifluoromethoxy group and a 1,1,2-trifluoro-2-chloroethoxy group.

8. The benzylideneaminoxyalkanoic acid amide derivative according to Claim 2, wherein said halo-lower alkylthio group as the substituent for $R^1$ is selected from the group consisting of a trifluoromethylthio group, a difluorochloromethylthio group and a difluorobromomethylthio group.

9. The benzylideneaminoxyalkanoic acid amide derivative according to Claim 2, wherein said $R^1$ is a phenyl group substituted by a chlorine atom, a fluorine atom, a methyl group, a methoxy group, a trifluoromethyl group, a trifluoromethoxy group, a cyano group or a nitro group, $R^2$ is an ethyl group, and $R^3$ is a phenyl group which may be substituted by a methyl group, a halogen atom or a trifluoromethyl group, or a thienyl group.

10. The benzylideneaminoxyalkanoic acid thioamide derivative according to Claim 1, wherein said derivative is represented by the formula:

$$R^1-CH=N-O-\underset{\underset{R^2}{|}}{CH}-\overset{\overset{S}{\|}}{C}-NH-(CH_2)_n-R^3 \qquad (I'')$$

wherein $R^1$, $R^2$, $R^3$ and n have the same meanings as defined in Claim 1.

**11.** The benzylideneaminoxyalkanoic acid thioamide derivative according to Claim 10, wherein said $R^1$ is a phenyl group substituted by a halogen atom, a halo-lower alkyl group, a halo-lower alkoxy group, a cyano group or a nitro group.

**12.** The benzylideneaminoxyalkanoic acid thioamide derivative according to Claim 10, wherein said $R^2$ is an alkyl group having 1 to 4 carbon atoms.

**13.** The benzylideneaminoxyalkanoic acid thioamide derivative according to Claim 10, wherein said $R^3$ is a phenyl group, which may be substituted by a chlorine or fluorine atom, or a substituted or unsubstituted thienyl group.

**14.** The benzylideneaminoxyalkanoic acid thioamide derivative according to Claim 10, wherein $R^1$ is a phenyl group substitued at 3-position by a chlorine atom, a bromine atom, a fluorine atom, an iodine atom, a trifluoromethyl group, a difluoromethyl group, a 1,1-difluoroethyl group, a 1,1,2,2-tetrafluoroethyl group, a pentafluoromethyl group, a difluoromethoxy group, a trifluoromethoxy group or a 1,1,2-trifluoro-2-chloroethoxy group; $R^2$ is an ethyl group; and $R^3$ is a phenyl group substituted at 2-position and/or 4-position by a chlorine atom, a bromine atom, a fluorine atom, or an iodine atom.

**15.** A process for preparing the benzylideneaminoxyalkanoic acid amide derivative according to Claim 1, which comprises reacting an oxime compound represented by the formula:

$$R^1\text{-CH=N-OH} \qquad (II)$$

wherein $R^1$ has the same meaning as defined in Claim 1, with 2-halogenoalkanoic acid amide represented by the formula:

$$X-\underset{\underset{R^2}{|}}{CH}-\overset{\overset{O}{\|}}{C}-NH-(CH_2)_n-R^3 \qquad (III)$$

wherein $R^2$, $R^3$ and n have the meanings defined in claim 1, and X represents a halogen atom.

**16.** A process for preparing the benzylideneaminoxyalkanoic acid amide derivative according to Claim 1, which comprises reacting a benzylideneaminoxyalkanoic acid halide represented by the formula:

$$R^1-CH=N-O-\underset{\underset{R^2}{|}}{CH}-\overset{\overset{O}{\|}}{C}-Y \qquad (IV)$$

wherein $R^1$ and $R^2$ have the same meanings as defined in Claim 1 and Y represents a halogen atom,

with an amine represented by the formula:

$$NH_2\text{-}(CH_2)_n\text{-}R^3 \qquad (V)$$

wherein $R^3$ and n have the same meanings as defined in Claim 1.

**17.** A process for preparing the benzylideneaminoxyalkanoic acid amide derivative according to Claim 1, which comprises reacting an aldehyde represented by the formula:

$$R^1\text{-CHO} \qquad (VI)$$

wherein $R^1$ has the same meaning as defined in Claim 1, with an aminoxyalkanoic acid amide represented by the formula:

$$NH_2-O-\underset{\underset{R^2}{|}}{CH}-\overset{\overset{O}{\|}}{C}-NH-(CH_2)_n-R^3 \qquad (VII)$$

wherein $R^2$, $R^3$ and n have the same meanings as defined in Claim 1.

**18.** A process for preparing the benzylideneaminoxyalkanoic acid amide derivative according to Claim 1, which comprises reacting a benzylideneamonoxyalkanoic acid ester represented by the formula:

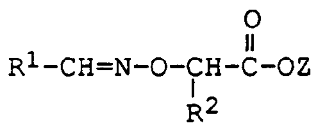

(VIII)

wherein $R^1$ and $R^2$ have the same meanings as defined in Claim 1, and Z represents a lower alkyl group,
with an amine represented by the formula:

$$NH_2\text{-}(CH_2)_n\text{-}R^3 \qquad (IX)$$

wherein $R^3$ and n have the same meanings as defined in Claim 1.

**19.** A process for preparing the benzylideneaminoxyalkanoic acid amide derivative according to Claim 10, which comprises reacting a compound represented by the formula:

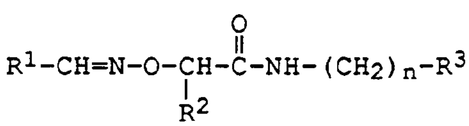

(I')

wherein $R^1$, $R^2$, $R^3$ and n have the same meanings as defined in Claim 1,
with a thiocarbonylation reagent.

**20.** A herbicide which comprises containing the benzylideneaminoxyalkanoic acid (thio)amide derivative as defined in Claim 1 as active ingredient.

**Claims for the following Contracting State : ES**

**1.** A process for preparing a benzylideneaminoxyalkanoic acid amide derivative represented by the following formula:

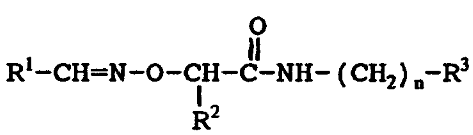

(I)

wherein n is an integer of 0 or 1, $R^1$ represents a phenyl group which may have one or two substituents selected from the group consisting of a lower alkyl group, a lower alkoxy group, a halogen atom, a halo-lower alkyl group, a halo-lower alkoxy group, a halo-lower alkylthio group, a halo-lower alkylsulfonyl group, a cyano group and a nitro group, or a thienyl group or a furyl group which may have a substituent selected from a lower alkyl group or a halogen atom; $R^2$ represents a lower alkyl group; and $R^3$ represents a phenyl group which may have one or two substituents selected from the group consisting of a lower alkyl group, a halogen atom, a halo-lower alkyl group and a lower alkoxy group, or a cycloalkyl group, a thienyl group or a furyl group
which comprises reacting an oxime compound represented by the formula:

$$R^1\text{-}CH\text{=}N\text{-}OH \qquad (II)$$

wherein $R^1$ has the same meaning as defined above, with 2-halogenoalkanoic acid amide represented by the formula:

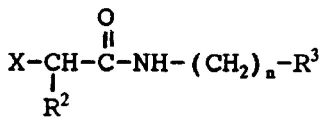

(III)

wherein $R^2$, $R^3$ and n have the meanings defined above, and X represents a halogen atom.

2. A process for preparing the benzylideneaminoxyalkanoic acid amide derivative as defined in Claim 1, which comprises reacting a benzylideneaminoxyalkanoic acid halide represented by the formula:

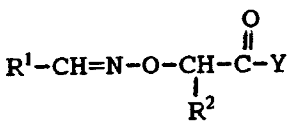

(IV)

wherein $R^1$ and $R^2$ have the same meanings as defined in Claim 1 and Y represents a halogen atom, with an amine represented by the formula:

$NH_2\text{-}(CH_2)_n\text{-}R^3$ (V)

wherein $R^3$ and n have the same meanings as defined in Claim 1.

3. A process for preparing the benzylideneaminoxyalkanoic acid amide derivative as defined in Claim 1, which comprises reacting an aldehyde represented by the formula:

$R^1\text{-}CHO$ (VI)

wherein $R^1$ has the same meaning as defined in Claim 1, with an aminoxyalkanoic acid amide represented by the formula:

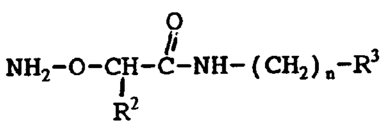

(VII)

wherein $R^2$, $R^3$ and n have the same meanings as defined in Claim 1.

4. A process for preparing the benzylideneaminoxyalkanoic acid amide derivative as defined in Claim 1, which comprises reacting a benzylideneaminoxyalkanoic acid ester represented by the formula:

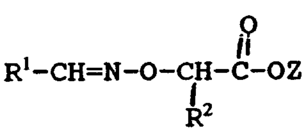

(VIII)

wherein $R^1$ and $R^2$ have the same meanings as defined in Claim 1, and Z represents a lower alkyl group, with an amine represented by the formula:

$NH_2\text{-}(CH_2)_n\text{-}R^3$ (IX)

wherein $R^3$ and n have the same meanings as defined in Claim 1.

5. A process according to any one of Claims 1 to 4, wherein said lower alkyl group as the substituent(s) for $R^1$ or $R^3$ is selected from the group consisting of a methyl group, an ethyl group, a propyl group, an isopropyl group, a butyl group and a t-butyl group.

6. A process according to any one of Claims 1 to 4, wherein said lower alkoxy group as the substituent(s) for $R^1$ or $R^3$ is selected from the group consisting of a methoxy group and an ethoxy group.

7. A process according to any one of Claims 1 to 4, wherein said halogen atom as the substituent(s) for $R^1$ or $R^3$ is selected from the group consisting of a chlorine atom, a bromine atom and a fluorine atom.

**8.** A process according to any one of Claims 1 to 4, wherein said halo-lower alkyl group as the substituent(s) for $R^1$ or $R^3$ is selected from the group consisting of a trifluoromethyl group, a difluoromethyl group, a 1,1-difluoroethyl group, a 1,1,2,2-tetrafluoroethyl group and a pentafluoroethyl group.

**9.** A process according to any one of Claims 1 to 4, wherein said halo-lower alkoxy group as the substituent(s) for $R^1$ or $R^3$ is selected from the group consisting of a difluoromethoxy group, a tri-fluoromethoxy group and a 1,1,2-trifluoro-2-chloroethoxy group.

**10.** A process according to any one of Claims 1 to 4, wherein said halo-lower alkyl-thio group as the substituent for $R^1$ is selected from the group consisting of a trifluoromethylthio group, a difluorochloromethylthio group and a difluorobromomethyl-thio group.

**11.** A process according to any one of Claims 1 to 4, wherein said $R^1$ is a phenyl group substituted by a chlorine atom, a fluorine atom, a methyl group, a methoxy group, a trifluoromethyl group, a trifluoromethoxy gruop, a cyano group or a nitro group, $R^2$ is an ethyl group, and $R^3$ is a phenyl group which may be substituted by a methyl group, a halogen atom or a trifluoromethyl group, or a thienyl group.

**12.** A process for preparing a benzylideneaminoxyalkanoic acid amide derivative of formula:

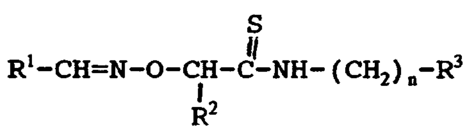

(I")

wherein $R^1$, $R^2$, $R^3$ and n have the same meanings as defined in Claim 1
which comprises reacting a compound represented by the formula:

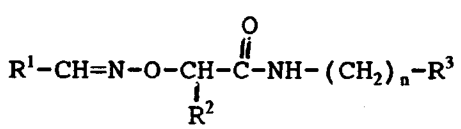

(I')

wherein $R^1$, $R^2$, $R^3$ and n have the same meanings as defined in Claim 1,
with a thiocarbonylation reagent.

**13.** A process according to Claim 12, wherein said $R^1$ is a phenyl group substituted by a halogen atom, a halo-lower alkyl group, a halo-lower alkoxy group, a cyano group, or a nitro group.

**14.** A process according to Claim 12, wherein said $R^2$ is an alkyl group having 1 to 4 carbon atoms.

**15.** A process according to Claim 12, wherein said $R^3$ is a phenyl group which may be substituted by a chlorine or fluorine atom, or a substituted or unsubstituted thienyl group.

**16.** A process according to Claim 12, wherein $R^1$ is a phenyl group substituted at 3-position by a chlorine atom, a bromine atom, a fluorine atom, an iodine atom, a trifluoromethyl group, a difluoromethyl group, a 1,1-difluoroethyl group, a 1,1,2,2-tetrafluoroethyl group, a pentafluoromethyl group, a difluoromethoxy group, a trifluoromethoxy group or a 1,1,2-trifluoro-2-chloroethoxy group; $R^2$ is an ethyl group; and $R^3$ is a phenyl group substituted at 2-position and/or 4-position by a chlorine atom, a bromine atom, a fluorine atom, or an iodine atom.

**17.** A process for the preparation of a herbicide which comprises mixing a compound prepared by a process as claimed in any one of Claims 1 to 16 with one or more of a carrier, surfactant, dispersing agent and auxiliary.

## Patentansprüche

### Patentansprüche für folgende Vertragsstaaten : DE, FR, GB, IT

**1.** Benzylidenaminoxyalkanoinsäure(thio)amid-Derivat, dargestellt durch die folgende Formel:

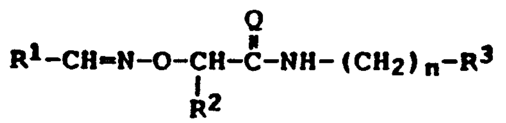

(I)

worin Q ein Sauerstoffatom oder ein Schwefelatom darstellt, n eine Ganzzahl von 0 oder 1 ist, und, wenn Q ein Sauerstoffatom ist, $R^1$ eine Phenylgruppe darstellt, die einen oder zwei Substituenten haben kann, ausgewählt von der Gruppe, bestehend aus einer niedrigeren Alkylgruppe, einer niedrigeren Alkoxygruppe, einem Halogenatom, einer Halo-niedrigere Alkylgruppe, einer Halo-niedrigere Alkoxygruppe, einer Halo-niedrigere Alkylthiogruppe, einer Halo-niedrigere Alkylsulfonylgruppe, einer Cyangruppe und einer Nitrogruppe, oder eine Thienylgruppe oder eine Furylgruppe, die einen Substituenten haben kann, ausgewählt von einer niedrigeren Alkylgruppe oder einem Halogenatom; $R^2$ eine niedrigere Alkylgruppe darstellt; und $R^3$ eine Phenylgruppe darstellt, die einen oder zwei Substituenten haben kann, ausgewählt von der Gruppe, bestehend aus einer niedrigeren Alkylgruppe, einem Halogenatom, einer Halo-niedrigere Alkylgruppe und einer niedrigeren Alkoxygruppe, oder eine Cycloalkylgruppe, eine Thienylgruppe oder eine Furylgruppe: und, wenn Q ein Schwefelatom darstellt, $R^1$ eine Phenylgruppe darstellt, die durch ein Halogenatom, eine Halo-niedrigere Alkylgruppe, eine Halo-niedrigere Alkoxygruppe, eine Cyangruppe oder eine Nitrogruppe substituiert sein kann; $R^2$ eine niedrigere Alkylgruppe darstellt, und $R^3$ eine Phenylgruppe darstellt, die durch ein Halogenatom oder eine Thienylgruppe substituiert sein kann.

**2.** Benzylidenaminoxyalkanoinsäureamid-Derivat nach Anspruch 1, bei welchem das Derivat dargestellt ist durch die Formel:

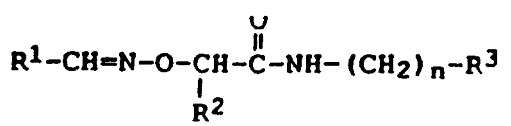

(I')

worin $R^1$, $R^2$, $R^3$ und n dieselben Bedeutungen haben wie angegeben im Anspruch 1.

**3.** Benzylidenaminoxyalkanoinsäureamid-Derivat nach Anspruch 2, bei welchem die niedrigere Alkylgruppe als Substituent für $R^1$ oder $R^3$ ausgewählt ist aus der Gruppe, bestehend aus einer Methylgruppe, einer Ethylgruppe, einer Propylgruppe, einer Isopropylgruppe, einer Butylgruppe und einer t-Butylgruppe.

**4.** Benzylidenaminoxyalkanoinsäureamid-Derivat nach Anspruch 2, bei welchem die niedrigere Alkoxygruppe als Substituent für $R^1$ oder $R^3$ ausgewählt ist aus der Gruppe, bestehend aus einer Methoxygruppe und einer Ethoxygruppe.

**5.** Benzylidenaminoxyalkanoinsäureamid-Derivat nach Anspruch 2, bei welchem das Halogenatom als Substituent für $R^1$ oder $R^3$ ausgewählt ist von der Gruppe, bestehend aus einem Chloratom, einem Bromatom und einem Fluoratom.

**6.** Benzylidenaminoxyalkanoinsäureamid-Derivat nach Anspruch 2, bei welchem die Halo-niedrigere Alkylgruppe als Substituent für $R^1$ oder $R^3$ ausgewählt ist von der Gruppe, bestehend aus einer Trifluormethylgruppe, einer Difluormethylgruppe, einer 1,1-Difluorethylgruppe, einer 1,1,2,2-Tetrafluorethylgruppe und einer Pentafluorethylgruppe.

**7.** Benzylidenaminoxyalkanoinsäureamid-Derivat nach Anspruch 2, bei welchem die Halo-niedrigere Alkoxygruppe als Substituent für $R^1$ oder $R^3$ ausgewählt ist von der Gruppe, bestehend aus einer Difluormethoxygruppe, einer Trifluormethoxygruppe und einer 1,1,2-Trifluor-2-Chlorethoxygruppe.

**8.** Benzylidenaminoxyalkanoinsäureamid-Derivat nach Anspruch 2, bei welchem die Halo-niedrigere Alkylthiogruppe als Substituent für $R^1$ ausgewählt ist von der Gruppe, bestehend aus einer Trifluormethylthiogruppe, einer Difluorchlormethylthiogruppe und einer Difluorbrommethylthiogruppe.

**9.** Benzylidenaminoxyalkanoinsäureamid-Derivat nach Anspruch 2, bei welchem $R^1$ eine Phenylgruppe ist, substituiert durch ein Chloratom, ein Fluoratom, eine Methylgruppe, eine Methoxygruppe, eine Trifluormethylgruppe, eine Trifluormethoxygruppe, eine Cyangruppe oder eine Nitrogruppe, $R^2$ eine Ethylgruppe ist und $R^3$ eine Phenylgruppe, die durch eine Methylgruppe, ein Halogenatom oder eine Trifluormethylgruppe substituiert sein kann, oder eine Thienylgruppe ist.

**10.** Benzylidenaminoxyalkanoinsäureamid-Derivat nach Anspruch 1, bei welchem das Derivat dargestellt ist durch die Formel:

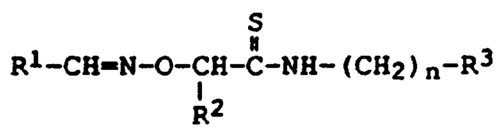
(I")

worin $R^1$, $R^2$, $R^3$ und n dieselben Bedeutungen haben wie angegeben im Anspruch 1.

**11.** Benzylidenaminoxyalkanoinsäurethioamid-Derivat nach Anspruch 10, bei welchem $R^1$ eine Phenylgruppe ist, substituiert durch ein Halogenatom, eine Halo-niedrigere Alkylgruppe, eine Halo-niedrigere Alkoxygruppe, eine Cyangruppe oder eine Nitrogruppe.

**12.** Benzylidenaminoxyalkanoinsäurethioamid-Derivat nach Anspruch 10, bei welchem $R^2$ eine Alkylgruppe mit 1 bis 4 Kohlenstoffatomen ist.

**13.** Benzylidenaminoxyalkanoinsäurethioamid-Derivat nach Anspruch 10, bei welchem $R^3$ eine Phenylgruppe, welche durch ein Chlor- oder Fluoratom substituiert sein kann, oder eine substituierte oder eine nicht substituierte Thienylgruppe ist.

**14.** Benzylidenaminoxyalkanoinsäurethioamid-Derivat nach Anspruch 10, bei welchem $R^1$ eine Phenylgruppe ist, substituiert an der 3-Position durch ein Chloratom, ein Bromatom, ein Fluoratom, ein Iodatom, eine Trifluormethylgruppe, eine Difluormethylgruppe, eine 1,1-Difluorethylgruppe, eine 1,1,2,2-Tetrafluorethylgruppe, eine Pentafluormethylgruppe, eine Difluormethoxygruppe, eine Trifluormethoxygruppe oder eine 1,1,2-Trifluor-2-Chlorethoxygruppe; $R^2$ eine Ethylgruppe ist; und $R^3$ eine Phenylgruppe ist, substituiert an der 2-Position und/oder and der 4-Position durch ein Chloratom, ein Bromatom, ein Fluoratom oder ein Iodatom.

**15.** Verfahren zum Herstellen des Benzylidenaminoxyalkanoinsäureamid-Derivats nach Anspruch 1, welches die Reaktion einer Oximverbindung, dargestellt durch die Formel:

$R^1$-CH=N-OH (II)

worin $R^1$ dieselbe Bedeutung hat wie angegeben im Anspruch 1, mit 2-Halogenalkanoinsäureamid umfaßt, dargestellt durch die Formel:

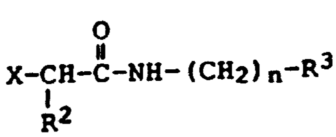
(III)

worin $R^2$, $R^3$ und n dieselben Bedeutungen haben wie angegeben im Anspruch 1 und X ein Halogenatom darstellt.

**16.** Verfahren zum Herstellen des Benzylidenaminoxyalkanoinsäureamid-Derivats nach Anspruch 1, welches die Reaktion eines Benzylidenaminoxyalkanoinsäure-Halids, dargestellt durch die Formel:

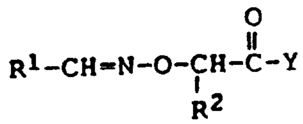

(IV)

worin $R^1$ und $R^2$ dieselben Bedeutungen haben wie angegeben im Anspruch 1 und Y ein Halogenatom darstellt,
mit einem Amin umfaßt, dargestellt durch die Formel:

$$NH_2\text{-}(CH_2)_n\text{-}R^3 \qquad (V)$$

worin $R^3$ und n dieselben Bedeutunge haben wie angegeben im Anspruch 1.

**17.** Verfahren zum Herstellen des Benzylidenaminoxyalkanoinsäureamid-Derivats nach Anspruch 1, welches die Reaktion eines Aldehyds, dargestellt durch die Formel:

$$R^1\text{-CHO} \qquad (VI)$$

worin $R^1$ dieselbe Bedeutung hat wie angegeben im Anspruch 1,
mit einem Aminoxyalkanoinsäureamid umfaßt, dargestellt durch die Formel:

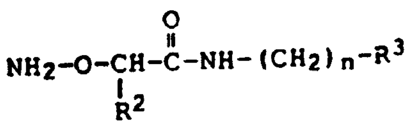

(VII)

worin $R^2$, $R^3$ und n dieselben Bedeutungen haben wie angegeben im Anspruch 1.

**18.** Verfahren zum Herstellen des Benzylidenaminoxyalkanoinsäureamid-Derivats nach Anspruch 1, welches die Reaktion eines Benzylidenaminoxyalkanoinsäure-Esters, dargestellt durch die Formel:

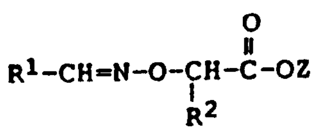

(VIII)

worin $R^1$ und $R^2$ dieselben Bedeutungen haben wie angegeben im Anspruch 1 und Z eine niedrige Alkylgruppe darstellt,
mit einem Amin umfaßt, dargestellt durch die Formel:

$$NH_2\text{-}(CH_2)_n\text{-}R^3 \qquad (IX)$$

worin $R^3$ und n dieselben Bedeutungen haben wie angegeben im Anspruch 1.

**19.** Verfahren zum Herstellen des Benzylidenaminoxyalkanoinsäureamid-Derivats nach Anspruch 10, welches die Reaktion einer Verbindung, dargestellt durch die Formel:

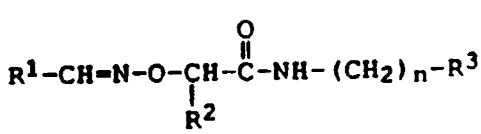

(I')

worin $R^1$, $R^2$, $R^3$ und n dieselben Bedeutungen haben wie angegeben im Anspruch 1,
mit einem Thiocarbonylation-Reagens umfaßt.

**20.** Herbizid, welches als einen aktiven Bestandteil das Benzylidenaminoxyalkanoinsäure(thio)amid-Derivat wie angegeben im Anspruch 1 enthält.

**Patentansprüche für folgenden Vertragsstaat : ES**

1. Verfahren zum Herstellen eines Benzylidenaminoxyalkanoinsäureamid-Derivats, dargestellt durch die folgende Formel:

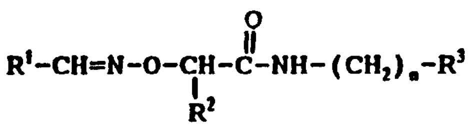

(I)

worin n eine Ganzzahl von 0 oder 1 ist, $R^1$ eine Phenylgruppe darstellt, die einen oder zwei Substituenten haben kann, ausgewählt von der Gruppe, bestehend aus einer niedrigeren Alkylgruppe, einer niedrigeren Alkoxygruppe, einem Halogenatom, einer Halo-niedrigeren Alkylgruppe, einer Halo-niedrigere Alkoxygruppe, einer Haloniedrigere Alkylthiogruppe, einer Halo-niedrigere Alkylsulfonylgruppe, einer Cyangruppe und einer Nitrogruppe, oder eine Thienylgruppe oder eine Furylgruppe, die einen Substituenten haben kann, ausgewählt von einer niedrigeren Alkylgruppe oder einem Halogenatom; $R^2$ eine niedrigere Alkylgruppe darstellt; und $R^3$ eine Phenylgruppe darstellt, die einen oder zwei Substituenten haben kann, ausgewählt von der Gruppe, bestehend aus einer niedrigeren Alkylgruppe, einem Halogenatom, einer Halo-niedrigere Alkylgruppe und einer niedrigeren Alkoxygruppe, oder eine Cycloalkylgruppe, eine Thienylgruppe oder eine Furylgruppe,
welches die Reaktion einer Oximverbindung, dargestellt durch die Formel:

$$R^1\text{-CH=N-OH} \qquad \text{(II)}$$

worin $R^1$ dieselbe Bedeutung hat wie vorstehend angegeben, mit 2-Halogenalkanoinsäureamid umfaßt, dargestellt durch die Formel:

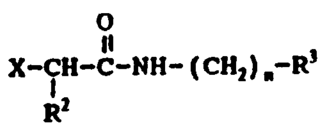

(III).

worin $R^2$, $R^3$ und n dieselben Bedeutungen haben wie vorstehend angegeben und X ein Halogenatom darstellt.

2. Verfahren zum Herstellen des Benzylidenaminoxyalkanoinsäureamid-Derivats nach Anspruch 1, welches die Reaktion eines Benzylidenaminoxyalkanoinsäure-Halids, dargestellt durch die Formel:

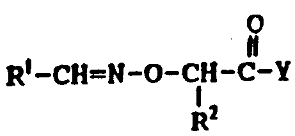

(IV)

worin $R^1$ und $R^2$ dieselben Bedeutungen haben wie angegeben im Anspruch 1 und Y ein Halogenatom darstellt,
mit einem Amin umfaßt, dargestellt durch die Formel:

$$NH_2\text{-}(CH_2)_n\text{-}R^3 \qquad \text{(V)}$$

worin $R^3$ und n dieselben Bedeutunge haben wie angegeben im Anspruch 1.

3. Verfahren zum Herstellen des Benzylidenaminoxyalkanoinsäureamid-Derivats nach Anspruch 1, welches die Reaktion eines Aldehyds, dargestellt durch die Formel:

$$R^1\text{-CHO} \qquad \text{(VI)}$$

worin $R^1$ dieselbe Bedeutung hat wie angegeben im Anspruch 1,
mit einem Aminoxyalkanoinsäureamid umfaßt, dargestellt durch die Formel:

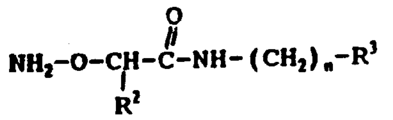 (VII)

worin $R^2$, $R^3$ und n dieselben Bedeutungen haben wie angegeben im Anspruch 1.

**4.** Verfahren zum Herstellen des Benzylidenaminoxyalkanoinsäureamid-Derivats nach Anspruch 1, welches die Reaktion eines Benzylidenaminoxyalkanoinsäure-Esters, dargestellt durch die Formel:

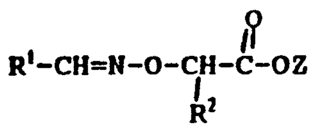 (VIII)

worin $R^1$ und $R^2$ dieselben Bedeutungen haben wie angegeben im Anspruch 1 und Z eine niedrige Alkylgruppe darstellt, mit einem Amin umfaßt, dargestellt durch die Formel:

$$NH_2\text{-}(CH_2)_n\text{-}R^3 \qquad \text{(IX)}$$

worin $R^3$ und n dieselben Bedeutungen haben wie angegeben im Anspruch 1.

**5.** Verfahren nach einem der Ansprüche 1 bis 4, bei welchem die niedrigere Alkylgruppe als Substituent für $R^1$ oder $R^3$ ausgewählt ist aus der Gruppe, bestehend aus einer Methylgruppe, einer Ethylgruppe, einer Propylgruppe, einer Isopropylgruppe, einer Butylgruppe und einer t-Butylgruppe.

**6.** Verfahren nach einem der Ansprüche 1 bis 4, bei welchem die niedrigere Alkoxygruppe als Substituent für $R^1$ oder $R^3$ ausgewählt ist aus der Gruppe, bestehend aus einer Methoxygruppe und einer Ethoxygruppe.

**7.** Verfahren nach einem der Ansprüche 1 bis 4, bei welchem das Halogenatom als Substituent für $R^1$ oder $R^3$ ausgewählt ist von der Gruppe, bestehend aus einem Chloratom, einem Bromatom und einem Fluoratom.

**8.** Verfahren nach einem der Ansprüche 1 bis 4, bei welchem die Halo-niedrigere Alkylgruppe als Substituent für $R^1$ oder $R^3$ ausgewählt ist von der Gruppe, bestehend aus einer Trifluormethylgruppe, einer Difluormethylgruppe, einer 1,1-Difluorethylgruppe, einer 1,1,2,2-Tetrafluorethylgruppe und einer Pentafluorethylgruppe.

**9.** Verfahren nach einem der Ansprüche 1 bis 4, bei welchem die Halo-niedrigere Alkoxygruppe als Substituent für $R^1$ oder $R^3$ ausgewählt ist von der Gruppe, bestehend aus einer Difluormethoxygruppe, einer Trifluormethoxygruppe und einer 1,1,2-Trifluor-2-Chlorethoxygruppe.

**10.** Verfahren nach einem der Ansprüche 1 bis 4, bei welchem die Halo-niedrigere Alkylthiogruppe als Substituent für $R^1$ ausgewählt ist von der Gruppe, bestehend aus einer Trifluormethylthiogruppe, einer Difluorchlormethylthiogruppe und einer Difluorbrommethylthiogruppe.

**11.** Verfahren nach einem der Ansprüche 1 bis 4, bei welchem $R^1$ eine Phenylgruppe ist, substituiert durch ein Chloratom, ein Fluoratom, eine Methylgruppe, eine Methoxygruppe, eine Trifluormethylgruppe, eine Trifluormethoxygruppe, eine Cyangruppe oder eine Nitrogruppe, $R^2$ eine Ethylgruppe ist, und $R^3$ eine Phenylgruppe, die durch eine Methylgruppe, ein Halogenatom oder eine Triffuormethylgruppe substituiert sein kann, oder eine Thienylgruppe ist.

**12.** Verfahren zum Herstellen eines Benzylidenaminoxyalkanoinsäureamid-Derivats der Formel:

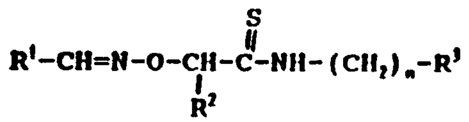

(I")

worin $R^1$, $R^2$, $R^3$ und n dieselben Bedeutungen haben wie angegeben im Anspruch 1, welches die Reaktion einer Verbindung, dargestellt durch die Formel:

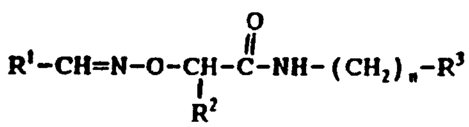

(I')

worin $R^1$, $R^2$, $R^3$ und n dieselben Bedeutungen haben wie angegeben im Anspruch 1, mit einem Thiocarbonylation-Reagens umfaßt.

13. Verfahren nach Anspruch 12, bei welchem $R^1$ eine Phenylgruppe ist, substituiert durch ein Halogenatom, eine Halo-niedrigere Alkylgruppe, eine Halo-niedrigere Alkoxygruppe, eine Cyangruppe oder eine Nitrogruppe.

14. Verfahren nach Anspruch 12, bei welchem $R^2$ eine Alkylgruppe mit 1 bis 4 Kohlenstoffatomen ist.

15. Verfahren nach Anspruch 12, bei welchem $R^3$ eine Phenylgruppe ist, die durch ein Chlor- oder Fluoratom substituiert sein kann, oder eine substituierte oder nicht substituierte Thienylgruppe.

16. Verfahren nach Anspruch 12, bei welchem $R^1$ eine Phenylgruppe ist, substituiert an der 3-Position durch ein Chloratom, ein Bromatom, ein Fluoratom, ein Iodatom, eine Trifluormethylgruppe, eine Difluormethylgruppe, eine 1,1-Difluorethylgruppe, eine 1,1,2,2-Tetrafluorethylgruppe, eine Pentafluormethylgruppe, eine Difluormethoxygruppe, eine Trifluormethoxygruppe oder eine 1,1,2-Trifluor-2-Chlorethoxygruppe; $R^2$ eine Ethylgruppe ist; und $R^3$ eine Phenylgruppe ist, substituiert an der 2-Positin und/oder an der 4-Position durch ein Chloratom, ein Bromatom, ein Fluoratom oder ein Iodatom.

17. Verfahren zum Herstellen eines Herbizids, welches das Mischen einer Verbindung, hergestellt durch ein Verfahren gemäß einem der Ansprüche 1 bis 16, mit einem oder mehreren eines Trägers, eines Tensids, eines Dispergiermittels und eines Hilfsstoffes umfaßt.

## Revendications

### Revendications pour les Etats contractants suivants : DE, FR, GB, IT

1. Dérivé (thio)amide d'acide benzylidèneaminoxyalcanoïque représenté par la formule suivante :

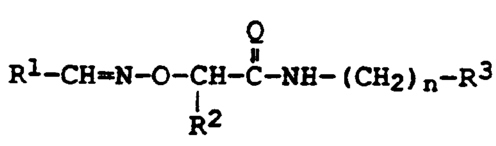

(I)

dans laquelle Q représente un atome d'oxygène ou un atome de soufre, n est un nombre 0 ou 1, et lorsque Q est un atome d'oxygène, $R^1$ représente un groupement phényle qui peut avoir un ou deux substituants choisis dans le groupe constitué par un groupement alkyle inférieur, un groupement alcoxy inférieur, un atome d'halogène, un groupement haloalkyle inférieur, un groupement haloalcoxy inférieur, un groupement haloalkylthio inférieur, un groupement haloalkylsulfonyle inférieur, un groupement cyano et un groupement nitro, ou un groupement thiényle ou un groupement furyle qui peuvent avoir un substituant choisi

parmi un groupement alkyle inférieur ou un atome d'halogène ; $R^2$ représente un groupement alkyle inférieur ; et $R^3$ représente un groupement phényle qui peut avoir un ou deux substituants choisis dans le groupe constitué par un groupement alkyle inférieur, un atome d'halogène, un groupement haloalkyle inférieur et un groupement alcoxy inférieur, ou un groupement cycloalkyle, un groupement thiényle ou un groupement furyle ; et lorsque Q est un atome de soufre, $R^1$ représente un groupement phényle qui peut être substitué par un atome d'halogène, un groupement haloalkyle inférieur, un groupement haloalcoxy inférieur, un groupement cyano ou un groupement nitro ; $R^2$ représente un groupement alkyle inférieur et $R^3$ représente un groupement phényle qui peut être substitué par un atome d'halogène ou un groupement thiényle.

2. Dérivé amide d'acide benzylidèneaminoxyalcanoïque selon la revendication 1, représenté par la formule :

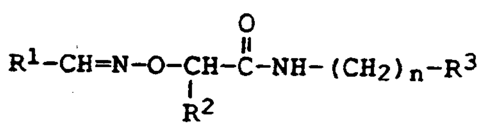

(I')

dans laquelle $R^1$, $R^2$, $R^3$ et n ont la signification donnée dans la revendication 1.

3. Dérivé amide d'acide benzylidèneaminoxyalcanoïque selon la revendication 2, dans lequel ledit groupement alkyle inférieur substituant de $R^1$ ou $R^3$ est choisi dans le groupe constitué par un groupement méthyle, un groupement éthyle, un groupement propyle, un groupement isopropyle, un groupement butyle et un groupement t-butyle.

4. Dérivé amide d'acide benzylidèneaminoxyalcanoïque selon la revendication 2, dans lequel ledit groupement alcoxy inférieur substituant de $R^1$ ou $R^3$ est choisi dans le groupe constitué par un groupement méthoxy et un groupement éthoxy.

5. Dérivé amide d'acide benzylidèneaminoxyalcanoïque selon la revendication 2, dans lequel l'atome d'halogène substituant de $R^1$ ou $R^3$ est choisi dans le groupe constitué par un atome de chlore, un atome de brome et un atome de fluor.

6. Dérivé amide d'acide benzylidèneaminoxyalcanoïque selon la revendication 2, dans lequel ledit groupement haloalkyle inférieur substituant de $R^1$ ou $R^3$ est choisi dans le groupe constitué par un groupement trifluorométhyle, un groupement difluorométhyle, un groupement 1,1-difluoroéthyle, un groupement 1,1,2,2-tétrafluoroéthyle et un groupement pentafluoroéthyle.

7. Dérivé amide d'acide benzylidèneaminoxyalcanoïque selon la revendication 2, dans lequel ledit groupement haloalcoxy inférieur substituant de $R^1$ ou $R^3$ est choisi dans le groupe constitué par un groupement difluorométhoxy, un groupement trifluorométhoxy et un groupement 1,1,2-trifluoro-2-chloroéthoxy.

8. Dérivé amide d'acide benzylidèneaminoxyalcanoïque selon la revendication 2, dans lequel ledit groupement haloalkylthio inférieur substituant de $R^1$ est choisi dans le groupe constitué par un groupement trifluorométhylthio, un groupement difluorochlorométhylthio et un groupement difluorobromométhylthio.

9. Dérivé amide d'acide benzylidèneaminoxyalcanoïque selon la revendication 2, dans lequel ledit $R^1$ est un groupement phényle substitué par un atome de chlore, un atome de fluor, un groupement méthyle, un groupement méthoxy, un groupement trifluorométhyle, un groupement trifluorométhoxy, un groupement cyano ou un groupement nitro, $R^2$ est un groupement éthyle, et $R^3$ est un groupement phényle qui peut être substitué par un groupement méthyle, un atome d'halogène ou un groupement trifluorométhyle, ou un groupement thiényle.

10. Dérivé thioamide d'acide benzylidèneaminoxyalcanoïque selon la revendication 1, représenté par la formule :

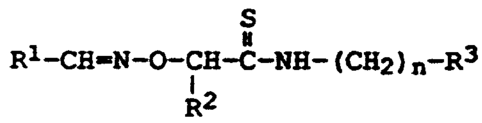

(I")

dans laquelle $R^1$, $R^2$, $R^3$ et n ont la même signification que dans la revendication 1.

**11.** Dérivé thioamide d'acide benzylidèneaminoxyalcanoïque selon la revendication 10, dans lequel $R^1$ est un groupement phényle substitué par un atome d'halogène, un groupement haloalkyle inférieur, un groupement haloalcoxy inférieur, un groupement cyano ou un groupement nitro.

**12.** Dérivé thioamide d'acide benzylidèneaminoxyalcanoïque selon la revendication 10, dans lequel $R^2$ est un groupement alkyle ayant de 1 à 4 atomes de carbone.

**13.** Dérivé thioamide d'acide benzylidèneaminoxyalcanoïque selon la revendication 10, dans lequel $R^3$ est un groupement phényle qui peut être substitué par un atome de chlore ou de fluor, ou un groupement thiényle substitué ou non.

**14.** Dérivé thioamide d'acide benzylidèneaminoxyalcanoïque selon la revendication 10, dans lequel $R^1$ est un groupement phényle substitué en position 3 par un atome de chlore, un atome de brome, un atome de fluor, un atome d'iode, un groupement trifluorométhyle, un groupement difluorométhyle, un groupement 1,1-difluoroéthyle, un groupement 1,1,2,2-tétrafluoroéthyle, un groupement pentafluoroéthyle, un groupement difluorométhoxy, un groupement trifluorométhoxy ou un groupement 1,1,2-trifluoro-2-chloroéthoxy ; $R^2$ est un groupement éthyle ; et $R^3$ est un groupement phényle substitué en position 2 et/ou en position 4 par un atome de chlore, un atome de brome, un atome de fluor ou un atome d'iode.

**15.** Procédé pour la préparation d'un dérivé amide d'acide benzylidèneaminoxyalcanoïque selon la revendication 1, qui comprend la réaction d'un composé oxime représenté par la formule $R^1$-CH=N-OH (II) dans laquelle $R^1$ a la même signification que dans la revendication 1,

avec un amide d'acide 2-halogénoalcanoïque représenté par la formule :

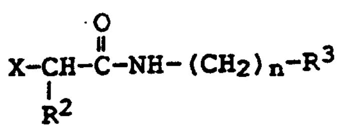

(III)

dans laquelle $R^2$, $R^3$ et n ont la même signification que dans la revendication 1, et X représente un atome d'halogène.

**16.** Procédé pour la préparation d'un dérivé amide d'acide benzylidèneaminoxyalcanoïque selon la revendication 1, qui comprend la réaction d'un halogénure d'acide benzylidèneaminoxyalcanoïque représenté par la formule :

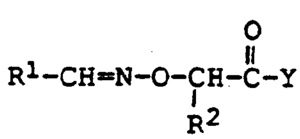

(IV)

dans laquelle $R^1$ et $R^2$ ont la même signification que dans la revendication 1, et X représente un atome d'halogène,

avec une amine représentée par la formule $NH_2$-$(CH_2)_n$-$R^3$ (V) dans laquelle $R^3$ et n ont la même signification que dans la revendication 1.

**17.** Procédé pour la préparation d'un dérivé amide d'acide benzylidèneaminoxyalcanoïque selon la revendication 1, qui comprend la réaction d'un aldéhyde représenté par la formule $R^1$-CHO (VI) dans laquelle $R^1$

a la même signification que dans la revendication 1, avec un amide d'acide aminoxyalcanoïque représenté par la formule :

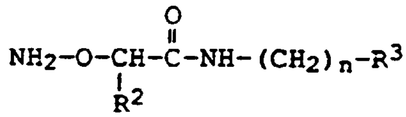 (VII)

dans laquelle $R^2$, $R^3$ et n ont la même signification que dans la revendication 1.

**18.** Procédé pour la préparation d'un dérivé amide d'acide benzylidèneaminoxyalcanoïque selon la revendication 1, qui comprend la réaction d'un ester d'acide benzylidèneaminoxyalcanoïque représenté par la formule :

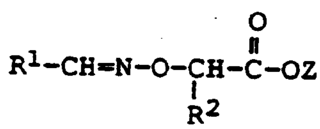 (VIII)

dans laquelle $R^1$ et $R^2$ ont la même signification que dans la revendication 1, et Z représente un groupement alkyle inférieur,

avec une amine représentée par la formule $NH_2-(CH_2)_n-R^3$ (IX) dans laquelle $R^3$ et n ont la même signification que dans la revendication 1.

**19.** Procédé pour la préparation d'un dérivé amide d'acide benzylidèneaminoxyalcanoïque selon la revendication 10, qui comprend la réaction d'un composé représenté par la formule :

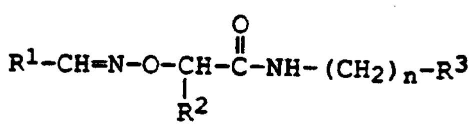 (I')

dans laquelle $R^1$, $R^2$, $R^3$ et n ont la même signification que dans la revendication 1,

avec un réactif de thiocarbonylation.

**20.** Herbicide comprenant le dérivé (thio)amide d'acide benzylidèneaminoxyalcanoïque tel que défini dans la revendication 1 comme ingrédient actif.

**Revendications pour l'Etat contractant suivant : ES**

**1.** Procédé pour la préparation d'un dérivé amide d'acide benzylidèneaminoxyalcanoïque représenté par la formule suivante :

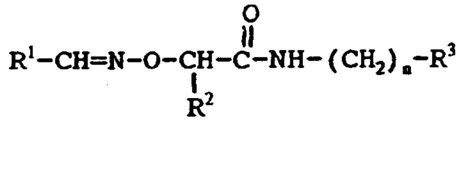 (I)

dans laquelle n est un nombre 0 ou 1, $R^1$ représente un groupement phényle qui peut avoir un ou deux substituants choisis dans le groupe constitué par un groupement alkyle inférieur, un groupement alcoxy inférieur, un atome d'halogène, un groupement haloalkyle inférieur, un groupement haloalcoxy inférieur, un groupement haloalkylthio inférieur, un groupement haloalkylsulfonyle inférieur, un groupement cyano et un groupement nitro, ou un groupement thiényle ou un groupement furyle qui peuvent avoir un substituant choisi parmi un groupement alkyle inférieur ou un atome d'halogène ; $R^2$ représente un groupement

alkyle inférieur ; et $R^3$ représente un groupement phényle qui peut avoir un ou deux substituants choisis dans le groupe constitué par un groupement alkyle inférieur, un atome d'halogène, un groupement haloalkyle inférieur et un groupement alcoxy inférieur, ou un groupement cycloalkyle, un groupement thiényle ou un groupement furyle,
qui comprend la réaction d'un composé oxime représenté par la formule $R^1$-CH=N-OH (II) dans laquelle $R^1$ a la signification donnée ci-dessus, avec un amide d'acide 2-halogénoalcanoïque représenté par la formule :

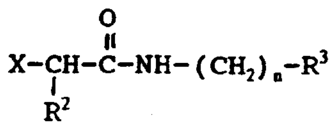

(III)

dans laquelle $R^2$, $R^3$ et n ont la signification donnée ci-dessus, et X représente un atome d'halogène.

2. Procédé pour la préparation d'un dérivé amide d'acide benzylidèneaminoxyalcanoïque tel que défini dans la revendication 1, qui comprend la réaction d'un halogénure d'acide benzylidèneaminoxyalcanoïque représenté par la formule :

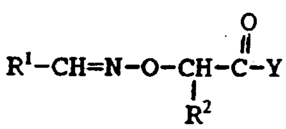

(IV)

dans laquelle $R^1$ et $R^2$ ont la même signification que dans la revendication 1, et Y représente un atome d'halogène, avec une amine représentée par la formule $NH_2$-$(CH_2)_n$-$R^3$ (V) dans laquelle $R^3$ et n ont la même signification que dans la revendication 1.

3. Procédé pour la préparation d'un dérivé amide d'acide benzylidèneaminoxyalcanoïque tel que défini dans la revendication 1, qui comprend la réaction d'un aldéhyde représenté par la formule $R^1$-CHO (VI) dans laquelle $R^1$ a la même signification que dans la revendication 1, avec un amide d'acide aminoxyalcanoïque représenté par la formule :

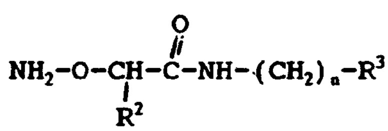

(VII)

dans laquelle $R^2$, $R^3$ et n ont la même signification que dans la revendication 1.

4. Procédé pour la préparation d'un dérivé amide d'acide benzylidèneaminoxyalcanoïque tel que défini dans la revendication 1, qui comprend la réaction d'un ester d'acide benzylidèneaminoxyalcanoïque représenté par la formule :

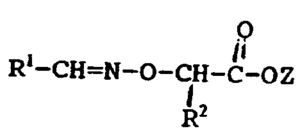

(VIII)

dans laquelle $R^1$ et $R^2$ ont la même signification que dans la revendication 1, et Z représente un groupement alkyle inférieur, avec une amine représentée par la formule $NH_2$-$(CH_2)_n$-$R^3$ (IX) dans laquelle $R^3$ et n ont la même signification que dans la revendication 1.

5. Procédé selon l'une quelconque des revendications 1 à 4, dans lequel ledit groupement alkyle inférieur substituant de $R^1$ ou $R^3$ est choisi dans le groupe constitué par un groupement méthyle, un groupement éthyle, un groupement propyle, un groupement isopropyle, un groupement butyle et un groupement t-butyle.

6. Procédé selon l'une quelconque des revendications 1 à 4, dans lequel ledit groupement alcoxy inférieur substituant de $R^1$ ou $R^3$ est choisi dans le groupe constitué par un groupement méthoxy et un groupement éthoxy.

7. Procédé selon l'une quelconque des revendications 1 à 4, dans lequel l'atome d'halogène substituant de $R^1$ ou $R^3$ est choisi dans le groupe constitué par un atome de chlore, un atome de brome et un atome de fluor.

8. Procédé selon l'une quelconque des revendications 1 à 4, dans lequel ledit groupement haloalkyle inférieur substituant de $R^1$ ou $R^3$ est choisi dans le groupe constitué par un groupement trifluorométhyle, un groupement difluorométhyle, un groupement 1,1-difluoroéthyle, un groupement 1,1,2,2-tétrafluoroéthyle et un groupement pentafluoroéthyle.

9. Procédé selon l'une quelconque des revendications 1 à 4, dans lequel ledit groupement haloalcoxy inférieur substituant de $R^1$ ou $R^3$ est choisi dans le groupe constitué par un groupement difluorométhoxy, un groupement trifluorométhoxy et un groupement 1,1,2-trifluoro-2-chloroéthoxy.

10. Procédé selon l'une quelconque des revendications 1 à 4, dans lequel ledit groupement haloalkylthio inférieur substituant de $R^1$ est choisi dans le groupe constitué par un groupement trifluorométhylthio, un groupement difluorochlorométhylthio et un groupement difluorobromométhylthio.

11. Procédé selon l'une quelconque des revendications 1 à 4, dans lequel ledit $R^1$ est un groupement phényle substitué par un atome de chlore, un atome de fluor, un groupement méthyle, un groupement méthoxy, un groupement trifluorométhyle, un groupement trifluorométhoxy, un groupement cyano ou un groupement nitro, $R^2$ est un groupement éthyle, et $R^3$ est un groupement phényle qui peut être substitué par un groupement méthyle, un atome d'halogène ou un groupement trifluorométhyle, ou un groupement thiényle.

12. Procédé pour la préparation d'un dérivé amide d'acide benzylidèneaminoxyalcanoïque répondant à la formule :

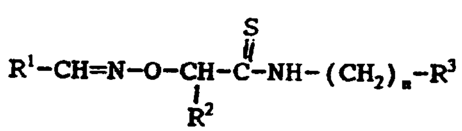

(I")

dans laquelle $R^1$, $R^2$, $R^3$ et n ont la signification donnée dans la revendication 1,
qui comprend la réaction d'un composé représenté par la formule :

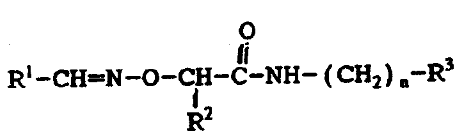

(I')

dans laquelle $R^1$, $R^2$, $R^3$ et n ont la signification donnée dans la revendication 1,
avec un agent de thiocarbonylation.

13. Procédé selon la revendication 12, dans lequel $R^1$ représente un groupement phényle substitué par un atome d'halogène, un groupement haloalkyle inférieur, un groupement haloalcoxy inférieur, un groupement cyano ou un groupement nitro.

**14.** Procédé selon la revendication 12, dans lequel $R^2$ représente un groupement alkyle ayant de 1 à 4 atomes de carbone.

**15.** Procédé selon la revendication 12, dans lequel $R^3$ représente un groupement phényle qui peut être substitué par un atome de chlore ou de fluor, ou un groupement thiényle substitué ou non.

**16.** Procédé selon la revendication 12, dans lequel $R^1$ est un groupement phényle substitué en position 3 par un atome de chlore, un atome de brome, un atome de fluor, un atome d'iode, un groupement trifluorométhyle, un groupement difluorométhyle, un groupement 1,1-difluoroéthyle, un groupement 1,1,2,2-tétrafluoroéthyle, un groupement pentafluoroéthyle, un groupement difluorométhoxy, un groupement trifluorométhoxy ou un groupement 1,1,2-trifluoro-2-chloroéthoxy ; $R^2$ est un groupement éthyle ; et $R^3$ est un groupement phényle substitué en position 2 et/ou en position 4 par un atome de chlore, un atome de brome, un atome de fluor ou un atome d'iode.

**17.** Procédé pour la préparation d'un herbicide dans lequel on mélange un composé préparé par un procédé tel que revendiqué dans l'une quelconque des revendications 1 à 16 avec un ou plusieurs constituants tels qu'une charge, un agent tensioactif, un agent dispersant ou un adjuvant.